# EUROPEAN PATENT APPLICATION

(11) **EP 0 628 572 A2**
(43) Date of publication of application: **14.12.1994**
(21) Application number: 94108256.2
(22) Date of filing: 27.05.1994
(51) Int. Cl.: C12N 15/51, C07K 13/00, C12P 21/08, C12Q 1/68, G01N 33/569

(54) **DNA originating in non-A non-B hepatitis virus gene and constituting polypeptide**

(30) Priority: 28.05.1993 JP 126709/93; 02.03.1994 JP 32201/94
(71) Applicant: Eisai Co., Ltd., Tokyo (JP); Arima, Terukatsu, Kagoshima (JP)
(72) Inventor: Aoyama, Muneo, Inashikigun, Ibaraki (JP); Obara, Takashi, Tsukuba-shi, Ibaraki (JP); Tohmatsu, Junichi, Tsuchiura-shi, Ibaraki (JP); Sawada, Takashi, Tsukuba-shi, Ibaraki (JP); Hosoda, Tetsuya, Tsukuba-shi, Ibaraki (JP); Iwasaki, Yoshihiro, Koto-ku, Tokyo (JP); Arima, Terukatsu, Kagoshima-shi, Kagoshima (JP)
(74) Representative: Kolb, Helga, Dr. Dipl.-Chem.

(57) **Abstract**

The present invention provides DNAs originating in non-A non-B hepatitis virus genome gene and polypeptides constituting said virus, which enable the diagnosis of hepatitis induced by said virus.

An RNA is extracted and purified from the plasma of a patient with non-A non-B hepatitis. By using this RNA as a template, a single strand DNA is prepared. Then, the DNA is amplified by the PCR method with the use of appropriately selected primers. The partial DNA sequence of the above-mentioned virus is identified by analyzing the structure and the constituting polypeptide of this virus is deduced. Thus, the antigen site is confirmed.

SEQUENCE LISTING

(2) INFORMATION FOR SEQ ID NO:1:

(i) SEQUENCE CHARACTERISTICS:

(A) LENGTH: 328 amino acids

(B) TYPE: amino acid

(C) STRANDEDNESS: single

(D) TOPOLOGY: linear

(ii) MOLECULE TYPE: peptide

(vi) ORIGINAL SOURCE:

(A) ORGANISM: non-A non-B hepatitis virus

(xi) SEQUENCE DESCRIPTION: SEQ ID NO:1:

Met Ser Thr Asn Pro Lys Pro Gln Arg Lys Thr Lys Arg Asn Thr Asn
1               5                   10                  15
Arg Arg Pro Gln Asp Val Lys Phe Pro Gly Gly Gly Gln Val Val Gly
            20                  25                  30
Gly Val Tyr Leu Leu Pro Arg Arg Gly Pro Arg Leu Gly Val Arg Ala
        35                  40                  45
Thr Arg Lys Thr Ser Glu Arg Ser Gln Pro Arg Glu Arg Arg Gln Pro
    50                  55                  60
Ile Pro Lys Asp Arg Arg Ser Thr Gly Lys Ser Trp Gly Lys Pro Gly
65                  70                  75                  80
Tyr Pro Trp Pro Leu Tyr Gly Asn Glu Gly Cys Gly Trp Ala Gly Trp
                85                  90                  95
Leu Leu Ser Pro Arg Gly Ser Arg Pro Thr Trp Gly Pro Thr Asp Pro
            100                 105                 110
Arg His Arg Ser Arg Asn Leu Gly Lys Val Ile Asp Thr Leu Thr Cys
        115                 120                 125

Gly Phe Ala Asp Leu Met Gly Tyr Ile Pro Val Val Gly Ala Pro Val
    130                 135                 140
Gly Gly Val Ala Arg Ala Leu Ala His Gly Val Arg Val Leu Glu Asp
145                 150                 155                 160
Gly Val Asn Tyr Ala Thr Gly Asn Leu Pro Gly Cys Ser Phe Ser Ile
                165                 170                 175
Phe Leu Leu Ala Leu Leu Ser Cys Val Thr Val Pro Val Ser Ala Val
            180                 185                 190
Glu Val Arg Asn Ile Ser Ser Ser Tyr Tyr Ala Thr Asn Asp Cys Ser
        195                 200                 205
Asn Asn Ser Ile Thr Trp Gln Leu Thr Asn Ala Val Leu His Leu Pro
    210                 215                 220
Gly Cys Val Pro Cys Glu Asn Asp Asn Gly Thr Leu Arg Cys Trp Ile
225                 230                 235                 240
Gln Val Thr Pro Asn Val Ala Val Lys Tyr Arg Gly Ala Leu Thr His
                245                 250                 255
Ser Leu Arg Thr His Val Asp Met Ile Val Met Ala Ala Thr Val Cys
            260                 265                 270
Ser Ala Leu Tyr Val Gly Asp Ala Cys Gly Ala Ile Met Ile Ala Ser
        275                 280                 285
Gln Ala Phe Ile Ile Ser Pro Glu Arg His Asn Phe Thr Gln Glu Cys
    290                 295                 300
Asn Cys Ser Ile Tyr Gln Gly Arg Ile Thr Gly His Arg Met Ala Trp
305                 310                 315                 320
Asp Met Met Leu Asn Trp Ser Pro
                325

(2) INFORMATION FOR SEQ ID NO:2:
(i) SEQUENCE CHARACTERISTICS:
(A) LENGTH: 1211 amino acids
(B) TYPE: amino acid
(C) STRANDEDNESS: single
(D) TOPOLOGY: linear
(ii) MOLECULE TYPE: peptide
(vi) ORIGINAL SOURCE:
(A) ORGANISM: non-A non-B hepatitis virus
(xi) SEQUENCE DESCRIPTION: SEQ ID NO:2:

Tyr Ala Ser Gln Gly Tyr Lys Val Leu Val Leu Asn Pro Ser Val Ala
1               5                   10                  15
Ala Thr Leu Gly Phe Gly Ala Tyr Met Ser Lys Ala His Gly Ile Asn
            20                  25                  30
Pro Asn Ile Arg Thr Gly Val Arg Thr Val Thr Thr Gly Asp Pro Ile
        35                  40                  45
Thr Tyr Ser Thr Tyr Gly Lys Phe Leu Ala Asp Gly Gly Cys Ser Ala
    50                  55                  60
Gly Ala Tyr Asp Ile Ile Ile Cys Asp Glu Cys His Ala Val Asp Ser
65                  70                  75                  80
Thr Thr Ile Leu Gly Ile Gly Thr Val Leu Asp Gln Ala Glu Thr Ala
                85                  90                  95
Gly Ile Arg Leu Val Val Leu Ala Thr Ala Thr Pro Pro Gly Thr Val
            100                 105                 110
Thr Thr Pro His Ser Asn Ile Glu Glu Val Ala Leu Ser His Glu Gly
        115                 120                 125
Glu Ile Pro Phe Tyr Gly Lys Ala Ile Pro Leu Ala Ser Ile Lys Gly
    130                 135                 140

Gly Arg His Leu Ile Phe Cys His Ser Lys Lys Lys Cys Asp Glu Leu
145                 150                 155                 160
Ala Ala Ala Leu Arg Gly Met Gly Val Asn Ala Val Ala Phe Tyr Arg
                165                 170                 175
Gly Leu Asp Val Ser Val Ile Pro Thr Gln Gly Asp Val Val Val Val
            180                 185                 190
Ala Thr Asp Ala Leu Met Thr Gly Tyr Thr Gly Asp Phe Asp Ser Val
        195                 200                 205
Ile Asp Cys Asn Val Arg Val Thr Gln Ile Val Asp Phe Ser Leu Asp
    210                 215                 220
Pro Thr Phe Thr Ile Thr Ile Gln Thr Val Pro Gln Asp Ala Val Ser
225                 230                 235                 240
Arg Ser Gln Arg Arg Gly Arg Thr Gly Arg Gly Arg Leu Gly Ile Tyr
                245                 250                 255
Arg Tyr Val Ser Ser Gly Glu Arg Pro Ser Gly Met Phe Asp Ser Ala
            260                 265                 270
Val Leu Cys Glu Cys Tyr Asp Ala Gly Ala Ala Trp Tyr Glu Leu Thr
        275                 280                 285
Pro Ala Glu Thr Thr Val Arg Leu Arg Ala Tyr Phe Asn Thr Pro Gly
    290                 295                 300
Leu Pro Val Cys Gln Asp His Leu Glu Phe Trp Glu Ala Val Phe Thr
305                 310                 315                 320
Gly Leu Thr His Ile Asp Ala His Phe Leu Ser Gln Thr Lys Gln Gly
                325                 330                 335
Gly Asp Asn Phe Ala Tyr Leu Thr Ala Tyr Gln Ala Thr Val Cys Ala
            340                 345                 350
Arg Ala Lys Ala Pro Pro Pro Ser Trp Asp Val Met Trp Lys Cys Leu
        355                 360                 365

Thr Arg Leu Lys Pro Thr Leu Thr Gly Pro Thr Pro Leu Leu Tyr Arg
    370                 375                 380
Leu Gly Ala Val Thr Asn Glu Val Thr Leu Thr His Pro Val Thr Lys
385                 390                 395                 400
Tyr Ile Ala Thr Cys Met Gln Ala Asp Leu Glu Ile Met Thr Ser Thr
                405                 410                 415
Trp Val Leu Ala Gly Gly Val Leu Ala Ala Val Ala Ala Tyr Cys Leu
            420                 425                 430
Ala Thr Gly Cys Ile Ser Ile Ile Gly Arg Leu His Leu Asn Asp Arg
        435                 440                 445
Val Val Val Ala Pro Asp Lys Glu Ile Leu Tyr Glu Ala Phe Asp Glu
    450                 455                 460
Met Glu Glu Cys Ala Ser Lys Ala Ala Leu Ile Glu Glu Gly Gln Arg
465                 470                 475                 480
Met Ala Glu Met Leu Lys Ser Lys Ile Gln Gly Leu Leu Gln Gln Ala
                485                 490                 495
Thr Lys Gln Ala Gln Asp Ile Gln Pro Ala Ile Gln Ser Ser Trp Pro
            500                 505                 510
Lys Leu Glu Gln Phe Trp Ala Lys His Met Trp Asn Phe Ile Ser Gly
        515                 520                 525
Ile Gln Tyr Leu Ala Gly Leu Ser Thr Leu Pro Gly Asn Pro Ala Val
    530                 535                 540
Ala Ser Met Met Ala Phe Ser Ala Ala Leu Thr Ser Pro Leu Pro Thr
545                 550                 555                 560
Ser Thr Thr Ile Leu Leu Asn Ile Met Gly Gly Trp Leu Ala Ser Gln
                565                 570                 575
Ile Ala Pro Pro Ala Gly Ala Thr Gly Phe Val Val Ser Gly Leu Val
            580                 585                 590

Gly Ala Ala Val Gly Ser Ile Gly Leu Pro Asn Ile Leu Val Asp Val
        595                 600                 605
Leu Ala Gly Tyr Gly Ala Gly Ile Ser Gly Ala Leu Val Ala Phe Lys
    610                 615                 620
Ile Met Ser Gly Glu Lys Pro Ser Val Glu Asp Val Val Asn Leu Leu
625                 630                 635                 640
Pro Ala Ile Leu Ser Pro Gly Ala Leu Val Val Gly Val Ile Cys Ala
                645                 650                 655
Ala Ile Leu Arg Arg His Ile Gly Gln Gly Glu Gly Ala Val Gln Trp
            660                 665                 670
Met Asn Arg Leu Ile Ala Phe Ala Ser Arg Gly Asn His Val Ala Pro
        675                 680                 685
Thr His Tyr Val Ala Glu Ser Asp Ala Ser Gln Arg Val Thr Gln Val
    690                 695                 700
Leu Ser Ser Leu Thr Ile Thr Ser Leu Leu Arg Arg Leu His Ala Trp
705                 710                 715                 720
Ile Thr Glu Asp Cys Pro Val Pro Cys Ser Gly Ser Trp Leu Arg Asp
                725                 730                 735
Ile Trp Asp Trp Val Cys Ser Ile Leu Thr Asp Phe Lys Asn Trp Leu
            740                 745                 750
Ser Ser Lys Leu Leu Pro Lys Met Pro Gly Leu Pro Phe Ile Ser Cys
        755                 760                 765
Gln Lys Gly Tyr Lys Gly Val Trp Ala Gly Thr Gly Val Met Thr Thr
    770                 775                 780
Arg Cys Pro Cys Gly Ala Asn Ile Ser Gly His Val Arg Met Gly Thr
785                 790                 795                 800
Met Arg Ile Thr Gly Pro Lys Thr Cys Leu Asn Leu Trp Gln Gly Thr
                805                 810                 815

Phe Pro Ile Asn Cys Tyr Thr Glu Gly Pro Cys Val Pro Lys Pro Pro
            820                 825                 830
Pro Asn Tyr Lys Thr Ala Ile Trp Arg Val Ala Ala Ser Glu Tyr Val
        835                 840                 845
Glu Ile Thr Arg His Gly Ser Phe Ser Tyr Val Thr Gly Leu Thr Thr
    850                 855                 860
Asp Asn Leu Lys Val Pro Cys Gln Val Pro Ala Pro Glu Phe Phe Ser
865                 870                 875                 880
Trp Val Asp Gly Val Gln Ile His Arg Phe Ala Pro Thr Pro Gly Pro
                885                 890                 895
Phe Phe Arg Asp Glu Val Ser Phe Thr Val Gly Leu Asn Ser Phe Val
            900                 905                 910
Val Gly Ser Gln Leu Pro Cys Asp Pro Glu Pro Asp Thr Glu Val Leu
        915                 920                 925
Ala Ser Met Leu Thr Asp Pro Ser His Ile Thr Ala Glu Ala Ala Ala
    930                 935                 940
Arg Arg Leu Ala Arg Gly Ser Pro Pro Ser Gln Ala Ser Ser Ser Ala
945                 950                 955                 960
Ser Gln Leu Ser Ala Pro Ser Leu Lys Ala Ile Cys Ala Ser His Lys
                965                 970                 975
Met Ala Tyr Asp Cys Asp Met Val Asp Ala Asn Leu Phe Met Gly Gly
            980                 985                 990
Asp Val Thr Arg Ile Glu Ser Asp Ser Lys Val Ile Val Leu Asp Tyr
        995                 1000                1005
Leu Asp Ser Met Thr Glu Thr Glu Asp Asp Arg Glu Pro Ser Ile Pro
    1010                1015                1020
Ser Glu Tyr Leu Ile Lys Arg Lys Arg Phe Pro Val Ala Val Pro Ser
1025                1030                1035                1040

Trp Ala His Pro Asp Tyr Asn Pro Pro Leu Val Glu Thr Trp Lys Arg
                1045                1050                1055
Pro Gly Tyr Glu Pro Pro Thr Val Leu Gly Cys Ala Leu Pro Pro Thr
            1060                1065                1070
Pro Gln Ala Pro Val Pro Pro Pro Arg Arg Arg Arg Ala Lys Val Leu
        1075                1080                1085
Thr Gln Asp Asn Val Glu Gly Val Leu Arg Glu Met Ala Asp Lys Val
    1090                1095                1100
Leu Ser Ser Leu Arg Gly Cys Asn Asp Thr Gly His Ser Thr Glu Met
1105                1110                1115                1120
Asp Thr Gly Gly Asp Ser Val Gln Gln Pro Ser Asp Glu Thr Thr Ala
                1125                1130                1135
Ser Glu Glu Gly Ser Leu Ser Ser Met Pro Pro Leu Glu Gly Glu Pro
            1140                1145                1150
Gly Asp Pro Asp Leu Glu Phe Gly Gln Ala Gly Ser Ala Pro Pro Ser
        1155                1160                1165
Glu Glu Glu Gly Glu Val Ile Asp Ser Asp Ser Lys Ser Trp Ser Thr
    1170                1175                1180
Val Ser Asp Gln Glu Asp Ala Val Ile Cys Cys Ser Met Ser Tyr Ser
1185                1190                1195                1200
Trp Thr Gly Ala Leu Ile Thr Pro Cys Gly Pro
                1205                1210

(2) INFORMATION FOR SEQ ID NO:3:
(i) SEQUENCE CHARACTERISTICS:
(A) LENGTH: 547 amino acids
(B) TYPE: amino acid
(D) TOPOLOGY: linear
(ii) MOLECULE TYPE: protein
(vi) ORIGINAL SOURCE:
(A) ORGANISM: non-A non-B hepatitis virus
(xi) SEQUENCE DESCRIPTION: SEQ ID NO:3:

Ala Ser Leu Arg Ala Lys Lys Val Thr Phe Asp Arg Val Gln Val Leu
1               5                   10                  15
Asp Ala His Tyr Asp Ser Val Leu Gln Asp Val Lys Arg Ala Ala Ser
            20                  25                  30
Lys Val Ser Ala Arg Leu Leu Ser Val Glu Glu Ala Cys Ala Leu Thr
        35                  40                  45
Pro Pro His Ser Ala Lys Ser Leu Tyr Gly Phe Gly Ala Lys Glu Val
    50                  55                  60
Arg Ser Leu Ser Arg Arg Ala Val Asn His Ile Arg Ser Val Trp Glu
65                  70                  75                  80
Asp Leu Leu Glu Asp Gln His Thr Pro Ile Asn Thr Thr Ile Met Ala
                85                  90                  95
Lys Asn Glu Val Phe Cys Ile Asp Pro Ala Lys Gly Gly Arg Lys Pro
            100                 105                 110
Ala Arg Leu Ile Val Tyr Pro Asp Leu Gly Val Arg Val Cys Glu Lys
        115                 120                 125
Met Ala Leu Tyr Asp Ile Ala Gln Lys Leu Pro Lys Ala Ile Met Gly
    130                 135                 140

Pro Ser Tyr Gly Phe Gln Tyr Ser Pro Ala Glu Arg Val Asp Phe Leu
145                 150                 155                 160
Leu Lys Ala Trp Gly Ser Lys Lys Asp Pro Met Gly Phe Ser Tyr Asp
                165                 170                 175
Thr Arg Cys Phe Asp Ser Thr Val Thr Glu Arg Asp Ile Arg Thr Glu
            180                 185                 190
Glu Ser Ile Tyr Gln Ala Cys Ser Leu Pro Gln Glu Ala Arg Thr Val
        195                 200                 205
Ile His Ser Leu Thr Glu Arg Leu Tyr Val Gly Gly Pro Met Thr Asn
    210                 215                 220
Ser Lys Gly Gln Ser Cys Gly Tyr Arg Arg Cys Arg Ala Ser Gly Val
225                 230                 235                 240
Phe Thr Thr Ser Met Gly Asn Thr Met Thr Cys Tyr Ile Lys Ala Leu
                245                 250                 255
Ala Ala Cys Lys Ala Ala Gly Ile Lys Asp Pro Ile Met Leu Val Cys
            260                 265                 270
Gly Asp Asp Leu Val Val Ile Ser Glu Ser Gln Gly Asn Glu Glu Asp
        275                 280                 285
Glu Arg Asn Leu Arg Ala Phe Thr Glu Ala Met Thr Arg Tyr Ser Ala
    290                 295                 300
Pro Pro Gly Asp Pro Pro Arg Pro Glu Tyr Asp Leu Glu Leu Ile Thr
305                 310                 315                 320
Ser Cys Ser Ser Asn Val Ser Val Ala Leu Asp Pro Arg Gly Arg Arg
                325                 330                 335
Arg Tyr Tyr Leu Thr Arg Asp Pro Thr Thr Pro Ile Ser Arg Ala Ala
            340                 345                 350
Trp Glu Thr Val Arg His Ser Pro Val Asn Ser Trp Leu Gly Asn Ile
        355                 360                 365

Ile Gln Tyr Ala Pro Thr Ile Trp Val Arg Met Val Ile Met Thr His
    370                 375                 380
Phe Phe Ala Ile Leu Leu Ala Gln Asp Thr Leu Asn Gln Asn Leu Asn
385                 390                 395                 400
Phe Glu Met Tyr Gly Ala Val Tyr Ser Val Asn Pro Leu Asp Leu Pro
                405                 410                 415
Ala Ile Ile Glu Arg Leu His Gly Leu Asp Ala Phe Ser Leu His Thr
            420                 425                 430
Tyr Ser Pro His Glu Leu Ser Arg Val Ala Ala Thr Leu Arg Lys Leu
        435                 440                 445
Gly Ala Pro Pro Leu Arg Ala Trp Lys Ser Arg Ala Arg Ala Val Arg
    450                 455                 460
Ala Ser Leu Ile Ala Gln Gly Gly Arg Ala Ala Ile Cys Gly Arg Tyr
465                 470                 475                 480
Leu Phe Asn Trp Ala Val Lys Thr Lys Leu Lys Leu Thr Pro Leu Pro
                485                 490                 495
Glu Ala Ala Arg Leu Asp Leu Ser Gly Trp Phe Thr Val Gly Ala Gly
            500                 505                 510
Gly Gly Asp Ile Phe His Ser Val Ser Arg Ala Arg Pro Arg Leu Leu
        515                 520                 525
Leu Leu Cys Leu Leu Leu Leu Ser Val Gly Val Gly Ile Phe Leu Leu
    530                 535                 540
Pro Ala Arg
545

(2) INFORMATION FOR SEQ ID NO:4:
(i) SEQUENCE CHARACTERISTICS:
(A) LENGTH: 1246 base pairs
(B) TYPE: nucleic acid
(C) STRANDEDNESS: double
(D) TOPOLOGY: linear
(ii) MOLECULE TYPE: cDNA to genomic RNA
(vi) ORIGINAL SOURCE:
(A) ORGANISM: non-A non-B hepatitis virus
(xi) SEQUENCE DESCRIPTION: SEQ ID NO:4:

TAGCCATGGC GTTAGTATGA GTGTCGTACA GCCTCCAGGC CCCCCCCTCC CGGGAGAGCC 60
ATAGTGGTCT GCGGAACCGG TGAGTACACC GGAATTGCCG GAAAGACTGG GTCCTTTCTT 120
GGATCAACCC ACTCTATGTC CGGTCATTTG GGCGTGCCCC CGCAAGACTG CTAGCCTAGT 180
AGCGTTGGGT TGCGAACGGC CTTGTGGTAC TGCCTGATAG GGTGCTTGCG AGTGCCCCGG 240
GAGGTCTCGT AGACCGTGCA TC ATG AGC ACA AAT CCT AAA CCT CAA AGA AAA 292
Met Ser Thr Asn Pro Lys Pro Gln Arg Lys
1               5                   10
ACC AAA AGA AAC ACA AAC CGC CGC CCA CAG GAC GTC AAG TTC CCG GGT 340
Thr Lys Arg Asn Thr Asn Arg Arg Pro Gln Asp Val Lys Phe Pro Gly
                15                  20                  25
GGC GGC CAG GTC GTT GGC GGA GTT TAC TTG CTG CCG CGC AGG GGC CCC 388
Gly Gly Gln Val Val Gly Gly Val Tyr Leu Leu Pro Arg Arg Gly Pro
            30                  35                  40
AGG TTG GGT GTG CGC GCG ACA AGG AAG ACT TCC GAG CGA TCC CAG CCG 436
Arg Leu Gly Val Arg Ala Thr Arg Lys Thr Ser Glu Arg Ser Gln Pro
        45                  50                  55

CGT GAG AGG CGC CAG CCC ATC CCG AAA GAT CGG CGC TCC ACC GGC AAG 484
Arg Glu Arg Arg Gln Pro Ile Pro Lys Asp Arg Arg Ser Thr Gly Lys
    60                  65                  70
TCC TGG GGA AAG CCA GGA TAC CCT TGG CCT CTG TAT GGA AAT GAG GGC 532
Ser Trp Gly Lys Pro Gly Tyr Pro Trp Pro Leu Tyr Gly Asn Glu Gly
75                  80                  85                  90
TGC GGC TGG GCA GGT TGG CTC CTG TCC CCC CGC GGG TCT CGT CCT ACA 580
Cys Gly Trp Ala Gly Trp Leu Leu Ser Pro Arg Gly Ser Arg Pro Thr
                95                  100                 105
TGG GGC CCC ACC GAC CCC CGG CAC AGA TCA CGC AAT TTG GGT AAG GTC 628
Trp Gly Pro Thr Asp Pro Arg His Arg Ser Arg Asn Leu Gly Lys Val
            110                 115                 120
ATC GAT ACC CTT ACG TGT GGT TTT GCC GAC CTC ATG GGG TAC ATC CCT 676
Ile Asp Thr Leu Thr Cys Gly Phe Ala Asp Leu Met Gly Tyr Ile Pro
        125                 130                 135
GTC GTT GGC GCC CCG GTC GGG GGC GTC GCT AGA GCT CTA GCA CAC GGT 724
Val Val Gly Ala Pro Val Gly Gly Val Ala Arg Ala Leu Ala His Gly
    140                 145                 150
GTT AGG GTC CTG GAA GAC GGG GTA AAT TAT GCA ACA GGG AAC CTG CCT 772
Val Arg Val Leu Glu Asp Gly Val Asn Tyr Ala Thr Gly Asn Leu Pro
155                 160                 165                 170
GGT TGC TCC TTT TCT ATC TTC TTA CTT GCT CTT CTG TCG TGC GTT ACA 820
Gly Cys Ser Phe Ser Ile Phe Leu Leu Ala Leu Leu Ser Cys Val Thr
                175                 180                 185
GTG CCA GTG TCT GCA GTG GAA GTC AGG AAC ATC AGT TCT AGC TAC TAC 868
Val Pro Val Ser Ala Val Glu Val Arg Asn Ile Ser Ser Ser Tyr Tyr
            190                 195                 200

GCC ACT AAT GAT TGC TCG AAC AAC AGC ATC ACC TGG CAG CTC ACT AAC 916
Ala Thr Asn Asp Cys Ser Asn Asn Ser Ile Thr Trp Gln Leu Thr Asn
        205                 210                 215
GCA GTT CTC CAC CTT CCT GGA TGC GTC CCA TGT GAG AAT GAT AAC GGC 964
Ala Val Leu His Leu Pro Gly Cys Val Pro Cys Glu Asn Asp Asn Gly
    220                 225                 230
ACC TTG CGA TGC TGG ATA CAA GTA ACA CCT AAT GTG GCT GTG AAA TAC 1012
Thr Leu Arg Cys Trp Ile Gln Val Thr Pro Asn Val Ala Val Lys Tyr
235                 240                 245                 250
CGC GGC GCA CTC ACC CAT AGC CTG CGA ACA CAT GTC GAC ATG ATC GTA 1060
Arg Gly Ala Leu Thr His Ser Leu Arg Thr His Val Asp Met Ile Val
                255                 260                 265
ATG GCA GCT ACG GTC TGC TCG GCC TTG TAT GTG GGG GAC GCG TGC GGG 1108
Met Ala Ala Thr Val Cys Ser Ala Leu Tyr Val Gly Asp Ala Cys Gly
            270                 275                 280
GCC ATA ATG ATC GCG TCA CAG GCT TTC ATA ATA TCG CCG GAA CGC CAC 1156
Ala Ile Met Ile Ala Ser Gln Ala Phe Ile Ile Ser Pro Glu Arg His
        285                 290                 295
AAC TTC ACC CAA GAG TGC AAC TGT TCC ATC TAT CAA GGC CGC ATC ACC 1204
Asn Phe Thr Gln Glu Cys Asn Cys Ser Ile Tyr Gln Gly Arg Ile Thr
    300                 305                 310
GGC CAC CGC ATG GCA TGG GAC ATG ATG CTA AAC TGG TCA CCA 1246
Gly His Arg Met Ala Trp Asp Met Met Leu Asn Trp Ser Pro
315                 320                 325

(2) INFORMATION FOR SEQ ID NO:5:
(i) SEQUENCE CHARACTERISTICS:
(A) LENGTH: 3633 base pairs
(B) TYPE: nucleic acid
(C) STRANDEDNESS: double
(D) TOPOLOGY: linear
(ii) MOLECULE TYPE: cDNA to genomic RNA
(vi) ORIGINAL SOURCE:
(A) ORGANISM: non-A non-B hepatitis virus
(xi) SEQUENCE DESCRIPTION: SEQ ID NO:5:

TAT GCC AGT CAG GGG TAT AAA GTA CTT GTA CTG AAT CCC TCT GTC GCG 48
Tyr Ala Ser Gln Gly Tyr Lys Val Leu Val Leu Asn Pro Ser Val Ala
1               5                   10                  15
GCT ACA CTC GGT TTT GGG GCC TAT ATG TCC AAA GCC CAC GGG ATT AAC 96
Ala Thr Leu Gly Phe Gly Ala Tyr Met Ser Lys Ala His Gly Ile Asn
            20                  25                  30
CCC AAC ATC AGA ACT GGA GTA CGG ACT GTG ACC ACC GGG GAC CCT ATC 144
Pro Asn Ile Arg Thr Gly Val Arg Thr Val Thr Thr Gly Asp Pro Ile
        35                  40                  45
ACC TAC TCC ACT TAT GGC AAG TTT CTC GCA GAT GGA GGC TGC TCG GCT 192
Thr Tyr Ser Thr Tyr Gly Lys Phe Leu Ala Asp Gly Gly Cys Ser Ala
    50                  55                  60
GGC GCC TAT GAC ATC ATC ATA TGC GAC GAA TGT CAT GCA GTG GAC TCT 240
Gly Ala Tyr Asp Ile Ile Ile Cys Asp Glu Cys His Ala Val Asp Ser
65                  70                  75                  80
ACT ACT ATC CTT GGC ATT GGA ACA GTC CTC GAC CAG GCT GAG ACC GCG 288
Thr Thr Ile Leu Gly Ile Gly Thr Val Leu Asp Gln Ala Glu Thr Ala
                85                  90                  95

GGT ATT AGG CTA GTG GTT TTG GCC ACA GCC ACA CCC CCT GGT ACA GTG 336
Gly Ile Arg Leu Val Val Leu Ala Thr Ala Thr Pro Pro Gly Thr Val
            100                 105                 110
ACA ACT CCC CAT AGT AAC ATA GAG GAG GTG GCT CTT AGT CAT GAA GGC 384
Thr Thr Pro His Ser Asn Ile Glu Glu Val Ala Leu Ser His Glu Gly
        115                 120                 125
GAG ATC CCC TTT TAC GGC AAG GCT ATT CCT CTA GCT TCC ATC AAA GGG 432
Glu Ile Pro Phe Tyr Gly Lys Ala Ile Pro Leu Ala Ser Ile Lys Gly
    130                 135                 140
GGC AGA CAC CTG ATC TTC TGC CAT TCA AAG AAG AAA TGC GAT GAA CTC 480
Gly Arg His Leu Ile Phe Cys His Ser Lys Lys Lys Cys Asp Glu Leu
145                 150                 155                 160
GCA GCA GCC CTT CGG GGC ATG GGT GTT AAT GCC GTT GCT TTC TAC AGG 528
Ala Ala Ala Leu Arg Gly Met Gly Val Asn Ala Val Ala Phe Tyr Arg
                165                 170                 175
GGT CTC GAC GTC TCC GTT ATA CCA ACC CAA GGG GAC GTG GTG GTA GTC 576
Gly Leu Asp Val Ser Val Ile Pro Thr Gln Gly Asp Val Val Val Val
            180                 185                 190
GCC ACC GAT GCC CTA ATG ACT GGA TAC ACC GGT GAC TTT GAC TCT GTC 624
Ala Thr Asp Ala Leu Met Thr Gly Tyr Thr Gly Asp Phe Asp Ser Val
        195                 200                 205
ATT GAC TGC AAC GTT CGA GTC ACT CAG ATT GTT GAC TTT AGC CTA GAT 672
Ile Asp Cys Asn Val Arg Val Thr Gln Ile Val Asp Phe Ser Leu Asp
    210                 215                 220
CCA ACT TTT ACC ATC ACC ATC CAA ACT GTC CCT CAA GAC GCT GTC TCC 720
Pro Thr Phe Thr Ile Thr Ile Gln Thr Val Pro Gln Asp Ala Val Ser
225                 230                 235                 240

CGT AGT CAA CGT AGA GGG AGA ACT GGG AGG GGG CGG CTG GGC ATT TAC 768
Arg Ser Gln Arg Arg Gly Arg Thr Gly Arg Gly Arg Leu Gly Ile Tyr
                245                 250                 255
AGG TAT GTC TCG TCA GGC GAA AGG CCG TCT GGG ATG TTC GAC AGC GCA 816
Arg Tyr Val Ser Ser Gly Glu Arg Pro Ser Gly Met Phe Asp Ser Ala
            260                 265                 270
GTG CTC TGC GAG TGC TAT GAT GCC GGG GCA GCC TGG TAC GAA CTC ACG 864
Val Leu Cys Glu Cys Tyr Asp Ala Gly Ala Ala Trp Tyr Glu Leu Thr
        275                 280                 285
CCT GCT GAG ACC ACA GTG AGA CTC CGG GCT TAT TTC AAC ACG CCC GGT 912
Pro Ala Glu Thr Thr Val Arg Leu Arg Ala Tyr Phe Asn Thr Pro Gly
    290                 295                 300
CTG CCC GTG TGT CAA GAC CAC TTG GAA TTC TGG GAG GCG GTC TTC ACA 960
Leu Pro Val Cys Gln Asp His Leu Glu Phe Trp Glu Ala Val Phe Thr
305                 310                 315                 320
GGT CTT ACA CAC ATT GAT GCC CAC TTC CTC TCC CAG ACG AAG CAA GGA 1008
Gly Leu Thr His Ile Asp Ala His Phe Leu Ser Gln Thr Lys Gln Gly
                325                 330                 335
GGA GAC AAC TTT GCG TAT CTA ACG GCC TAC CAG GCC ACA GTA TGC GCC 1056
Gly Asp Asn Phe Ala Tyr Leu Thr Ala Tyr Gln Ala Thr Val Cys Ala
            340                 345                 350
AGG GCA AAG GCC CCT CCA CCT TCG TGG GAC GTG ATG TGG AAG TGT CTA 1104
Arg Ala Lys Ala Pro Pro Pro Ser Trp Asp Val Met Trp Lys Cys Leu
        355                 360                 365
ACT AGG CTC AAA CCT ACA CTG ACT GGT CCT ACC CCC CTC CTG TAC CGC 1152
Thr Arg Leu Lys Pro Thr Leu Thr Gly Pro Thr Pro Leu Leu Tyr Arg
    370                 375                 380

TTG GGT GCC GTG ACC AAC GAG GTC ACC CTG ACG CAC CCT GTG ACG AAA 1200
Leu Gly Ala Val Thr Asn Glu Val Thr Leu Thr His Pro Val Thr Lys
385                 390                 395                 400
TAC ATC GCC ACG TGC ATG CAG GCT GAC CTT GAG ATC ATG ACA AGC ACA 1248
Tyr Ile Ala Thr Cys Met Gln Ala Asp Leu Glu Ile Met Thr Ser Thr
                405                 410                 415
TGG GTT CTG GCA GGG GGG GTG CTA GCT GCC GTG GCA GCT TAC TGC CTG 1296
Trp Val Leu Ala Gly Gly Val Leu Ala Ala Val Ala Ala Tyr Cys Leu
            420                 425                 430
GCG ACT GGC TGC ATT TCC ATC ATT GGC CGC CTA CAC CTG AAC GAT CGG 1344
Ala Thr Gly Cys Ile Ser Ile Ile Gly Arg Leu His Leu Asn Asp Arg
        435                 440                 445
GTG GTT GTG GCC CCT GAC AAG GAG ATC TTA TAC GAG GCC TTT GAT GAG 1392
Val Val Val Ala Pro Asp Lys Glu Ile Leu Tyr Glu Ala Phe Asp Glu
    450                 455                 460
ATG GAA GAG TGC GCC TCC AAA GCC GCC CTC ATT GAG GAA GGG CAG AGG 1440
Met Glu Glu Cys Ala Ser Lys Ala Ala Leu Ile Glu Glu Gly Gln Arg
465                 470                 475                 480
ATG GCG GAG ATG CTC AAG TCC AAA ATA CAA GGC CTC CTA CAA CAG GCT 1488
Met Ala Glu Met Leu Lys Ser Lys Ile Gln Gly Leu Leu Gln Gln Ala
                485                 490                 495
ACA AAA CAG GCC CAA GAC ATA CAG CCA GCC ATA CAG TCA TCA TGG CCC 1536
Thr Lys Gln Ala Gln Asp Ile Gln Pro Ala Ile Gln Ser Ser Trp Pro
            500                 505                 510
AAG CTC GAA CAA TTC TGG GCT AAG CAT ATG TGG AAC TTC ATC AGT GGC 1584
Lys Leu Glu Gln Phe Trp Ala Lys His Met Trp Asn Phe Ile Ser Gly
        515                 520                 525

ATA CAG TAC CTG GCC GGG CTC TCT ACC CTA CCG GGA AAT CCC GCG GTG 1632
Ile Gln Tyr Leu Ala Gly Leu Ser Thr Leu Pro Gly Asn Pro Ala Val
    530                 535                 540
GCA TCA ATG ATG GCC TTC AGC GCC GCG TTG ACC AGC CCA TTG CCC ACC 1680
Ala Ser Met Met Ala Phe Ser Ala Ala Leu Thr Ser Pro Leu Pro Thr
545                 550                 555                 560
AGC ACC ACG ATC CTC TTG AAC ATC ATG GGA GGA TGG TTG GCC TCT CAG 1728
Ser Thr Thr Ile Leu Leu Asn Ile Met Gly Gly Trp Leu Ala Ser Gln
                565                 570                 575
ATT GCC CCC CCT GCC GGA GCC ACT GGC TTC GTT GTC AGT GGT CTA GTA 1776
Ile Ala Pro Pro Ala Gly Ala Thr Gly Phe Val Val Ser Gly Leu Val
            580                 585                 590
GGG GCG GCC GTC GGG AGT ATA GGT CTA CCT AAC ATA CTG GTG GAC GTC 1824
Gly Ala Ala Val Gly Ser Ile Gly Leu Pro Asn Ile Leu Val Asp Val
        595                 600                 605
CTG GCC GGG TAC GGT GCA GGC ATC TCA GGG GCC CTC GTA GCT TTT AAG 1872
Leu Ala Gly Tyr Gly Ala Gly Ile Ser Gly Ala Leu Val Ala Phe Lys
    610                 615                 620
ATC ATG AGC GGC GAG AAG CCC TCG GTA GAA GAT GTT GTG AAT CTC CTG 1920
Ile Met Ser Gly Glu Lys Pro Ser Val Glu Asp Val Val Asn Leu Leu
625                 630                 635                 640
CCT GCT ATT TTG TCT CCT GGC GCT CTG GTG GTG GGA GTC ATC TGT GCA 1968
Pro Ala Ile Leu Ser Pro Gly Ala Leu Val Val Gly Val Ile Cys Ala
                645                 650                 655
GCG ATC TTG CGC CGC CAC ATC GGT CAG GGA GAG GGG GCG GTT CAG TGG 2016
Ala Ile Leu Arg Arg His Ile Gly Gln Gly Glu Gly Ala Val Gln Trp
            660                 665                 670

ATG AAT AGA CTG ATC GCT TTT GCC TCT AGG GGA AAC CAT GTT GCC CCT 2064
Met Asn Arg Leu Ile Ala Phe Ala Ser Arg Gly Asn His Val Ala Pro
        675                 680                 685
ACC CAC TAC GTG GCA GAG TCT GAC GCC TCT CAG CGC GTG ACG CAA GTG 2112
Thr His Tyr Val Ala Glu Ser Asp Ala Ser Gln Arg Val Thr Gln Val
    690                 695                 700
TTG AGT TCA CTC ACA ATT ACC AGC TTA CTT AGG AGA CTA CAT GCC TGG 2160
Leu Ser Ser Leu Thr Ile Thr Ser Leu Leu Arg Arg Leu His Ala Trp
705                 710                 715                 720
ATC ACT GAA GAT TGC CCA GTC CCT TGC TCG GGG TCT TGG CTC CGG GAC 2208
Ile Thr Glu Asp Cys Pro Val Pro Cys Ser Gly Ser Trp Leu Arg Asp
                725                 730                 735
ATT TGG GAT TGG GTT TGT TCC ATT CTC ACA GAC TTC AAG AAC TGG CTG 2256
Ile Trp Asp Trp Val Cys Ser Ile Leu Thr Asp Phe Lys Asn Trp Leu
            740                 745                 750
TCT TCA AAA CTG CTC CCC AAA ATG CCT GGC CTC CCC TTT ATC TCT TGT 2304
Ser Ser Lys Leu Leu Pro Lys Met Pro Gly Leu Pro Phe Ile Ser Cys
        755                 760                 765
CAA AAG GGG TAT AAG GGT GTG TGG GCC GGC ACG GGA GTC ATG ACC ACT 2352
Gln Lys Gly Tyr Lys Gly Val Trp Ala Gly Thr Gly Val Met Thr Thr
    770                 775                 780
CGA TGT CCA TGT GGA GCA AAC ATC TCG GGC CAT GTC CGC ATG GGC ACT 2400
Arg Cys Pro Cys Gly Ala Asn Ile Ser Gly His Val Arg Met Gly Thr
785                 790                 795                 800
ATG AGA ATA ACA GGC CCA AAG ACC TGC TTG AAC TTG TGG CAG GGG ACC 2448
Met Arg Ile Thr Gly Pro Lys Thr Cys Leu Asn Leu Trp Gln Gly Thr
                805                 810                 815

TTC CCC ATC AAT TGT TAC ACA GAA GGG CCT TGT GTG CCA AAA CCT CCT 2496
Phe Pro Ile Asn Cys Tyr Thr Glu Gly Pro Cys Val Pro Lys Pro Pro
            820                 825                 830
CCT AAT TAT AAG ACC GCA ATT TGG AGG GTG GCA GCG TCG GAG TAC GTT 2544
Pro Asn Tyr Lys Thr Ala Ile Trp Arg Val Ala Ala Ser Glu Tyr Val
        835                 840                 845
GAG ATC ACA CGG CAT GGC TCC TTC TCG TAC GTA ACG GGG TTG ACC ACT 2592
Glu Ile Thr Arg His Gly Ser Phe Ser Tyr Val Thr Gly Leu Thr Thr
    850                 855                 860
GAC AAC CTT AAG GTC CCT TGC CAG GTA CCA GCT CCA GAA TTC TTC TCT 2640
Asp Asn Leu Lys Val Pro Cys Gln Val Pro Ala Pro Glu Phe Phe Ser
865                 870                 875                 880
TGG GTG GAT GGG GTG CAG ATA CAC CGA TTC GCC CCC ACT CCA GGT CCC 2688
Trp Val Asp Gly Val Gln Ile His Arg Phe Ala Pro Thr Pro Gly Pro
                885                 890                 895
TTC TTT CGG GAT GAG GTA TCG TTC ACT GTG GGC CTC AAT TCC TTT GTG 2736
Phe Phe Arg Asp Glu Val Ser Phe Thr Val Gly Leu Asn Ser Phe Val
            900                 905                 910
GTC GGT TCT CAG CTC CCC TGC GAC CCT GAG CCG GAC ACT GAG GTG TTG 2784
Val Gly Ser Gln Leu Pro Cys Asp Pro Glu Pro Asp Thr Glu Val Leu
        915                 920                 925
GCC TCC ATG CTG ACA GAT CCG TCC CAC ATT ACA GCG GAG GCG GCA GCT 2832
Ala Ser Met Leu Thr Asp Pro Ser His Ile Thr Ala Glu Ala Ala Ala
    930                 935                 940
AGG CGA TTG GCC AGG GGA TCT CCC CCT TCA CAG GCC AGC TCT TCA GCA 2880
Arg Arg Leu Ala Arg Gly Ser Pro Pro Ser Gln Ala Ser Ser Ser Ala
945                 950                 955                 960

AGC CAG CTC TCC GCC CCG TCT TTG AAG GCT ATC TGT GCC AGC CAC AAG 2928
Ser Gln Leu Ser Ala Pro Ser Leu Lys Ala Ile Cys Ala Ser His Lys
                965                 970                 975
ATG GCA TAT GAT TGT GAC ATG GTG GAT GCA AAC CTT TTC ATG GGA GGT 2976
Met Ala Tyr Asp Cys Asp Met Val Asp Ala Asn Leu Phe Met Gly Gly
            980                 985                 990
GAT GTG ACC CGG ATC GAG TCT GAC TCT AAG GTG ATT GTT CTC GAC TAT 3024
Asp Val Thr Arg Ile Glu Ser Asp Ser Lys Val Ile Val Leu Asp Tyr
        995                 1000                1005
CTC GAT TCT ATG ACC GAG ACA GAG GAC GAT CGT GAG CCT TCA ATA CCA 3072
Leu Asp Ser Met Thr Glu Thr Glu Asp Asp Arg Glu Pro Ser Ile Pro
    1010                1015                1020
TCA GAG TAT TTG ATT AAG AGG AAA AGG TTC CCA GTG GCA GTA CCT TCC 3120
Ser Glu Tyr Leu Ile Lys Arg Lys Arg Phe Pro Val Ala Val Pro Ser
1025                1030                1035                1040
TGG GCC CAC CCA GAC TAT AAC CCC CCT TTG GTT GAG ACA TGG AAG AGG 3168
Trp Ala His Pro Asp Tyr Asn Pro Pro Leu Val Glu Thr Trp Lys Arg
                1045                1050                1055
CCG GGC TAT GAA CCA CCC ACT GTC CTA GGC TGT GCC CTT CCC CCC ACG 3216
Pro Gly Tyr Glu Pro Pro Thr Val Leu Gly Cys Ala Leu Pro Pro Thr
            1060                1065                1070
CCT CAA GCG CCA GTG CCC CCA CCT CGG AGG CGC CGT GCC AAA GTC CTG 3264
Pro Gln Ala Pro Val Pro Pro Pro Arg Arg Arg Arg Ala Lys Val Leu
        1075                1080                1085
ACT CAG GAC AAT GTG GAG GGG GTT CTC AGG GAG ATG GCG GAC AAA GTG 3312
Thr Gln Asp Asn Val Glu Gly Val Leu Arg Glu Met Ala Asp Lys Val
    1090                1095                1100

CTC AGC TCT CTC CGG GGT TGC AAT GAC ACC GGT CAC TCC ACT GAG ATG 3360
Leu Ser Ser Leu Arg Gly Cys Asn Asp Thr Gly His Ser Thr Glu Met
1105                1110                1115                1120
GAT ACC GGG GGA GAC AGC GTC CAG CAG CCC TCT GAC GAG ACT ACT GCT 3408
Asp Thr Gly Gly Asp Ser Val Gln Gln Pro Ser Asp Glu Thr Thr Ala
                1125                1130                1135
TCA GAA GAA GGA TCA CTG TCC TCC ATG CCC CCC CTT GAG GGA GAG CCG 3456
Ser Glu Glu Gly Ser Leu Ser Ser Met Pro Pro Leu Glu Gly Glu Pro
            1140                1145                1150
GGG GAC CCT GAC CTG GAG TTC GGA CAA GCC GGC TCC GCT CCC CCT TCC 3504
Gly Asp Pro Asp Leu Glu Phe Gly Gln Ala Gly Ser Ala Pro Pro Ser
        1155                1160                1165
GAG GAG GAG GGC GAG GTC ATT GAT TCG GAC TCT AAG TCG TGG TCT ACA 3552
Glu Glu Glu Gly Glu Val Ile Asp Ser Asp Ser Lys Ser Trp Ser Thr
    1170                1175                1180
GTC TCT GAT CAA GAG GAT GCT GTT ATT TGT TGC TCC ATG TCA TAC TCC 3600
Val Ser Asp Gln Glu Asp Ala Val Ile Cys Cys Ser Met Ser Tyr Ser
1185                1190                1195                1200
TGG ACA GGG GCC CTC ATA ACA CCG TGT GGG CCC 3633
Trp Thr Gly Ala Leu Ile Thr Pro Cys Gly Pro
                1205                1210

(2) INFORMATION FOR SEQ ID NO:6:
(i) SEQUENCE CHARACTERISTICS:
(A) LENGTH: 1696 base pairs
(B) TYPE: nucleic acid
(C) STRANDEDNESS: double
(D) TOPOLOGY: linear
(ii) MOLECULE TYPE: cDNA to genomic RNA
(vi) ORIGINAL SOURCE:
(A) ORGANISM: non-A non-B hepatitis virus
(xi) SEQUENCE DESCRIPTION: SEQ ID NO:6:

GCT TCT CTG AGG GCA AAG AAG GTG ACC TTT GAC AGG GTG CAG GTG CTG 48
Ala Ser Leu Arg Ala Lys Lys Val Thr Phe Asp Arg Val Gln Val Leu
1               5                   10                  15
GAC GCA CAC TAC GAC TCA GTC TTG CAG GAC GTT AAG CGA GCC GCC TCT 96
Asp Ala His Tyr Asp Ser Val Leu Gln Asp Val Lys Arg Ala Ala Ser
            20                  25                  30
AAG GTT AGT GCA AGG CTC CTC TCA GTA GAG GAA GCC TGC GCG CTG ACC 144
Lys Val Ser Ala Arg Leu Leu Ser Val Glu Glu Ala Cys Ala Leu Thr
        35                  40                  45
CCA CCC CAC TCC GCT AAA TCA CTA TAC GGA TTT GGG GCA AAA GAG GTG 192
Pro Pro His Ser Ala Lys Ser Leu Tyr Gly Phe Gly Ala Lys Glu Val
    50                  55                  60
CGC AGC TTA TCC AGG AGG GCC GTT AAC CAC ATC CGG TCC GTG TGG GAG 240
Arg Ser Leu Ser Arg Arg Ala Val Asn His Ile Arg Ser Val Trp Glu
65                  70                  75                  80
GAC CTC CTG GAA GAC CAA CAT ACT CCA ATT AAC ACA ACC ATC ATG GCC 288
Asp Leu Leu Glu Asp Gln His Thr Pro Ile Asn Thr Thr Ile Met Ala
                85                  90                  95

AAA AAT GAG GTG TTC TGT ATT GAC CCC GCT AAG GGC GGG AGG AAG CCA 336
Lys Asn Glu Val Phe Cys Ile Asp Pro Ala Lys Gly Gly Arg Lys Pro
            100                 105                 110
GCT CGC CTC ATC GTA TAC CCC GAT CTT GGG GTC AGG GTG TGC GAA AAG 384
Ala Arg Leu Ile Val Tyr Pro Asp Leu Gly Val Arg Val Cys Glu Lys
        115                 120                 125
ATG GCC CTC TAT GAC ATT GCA CAA AAG CTC CCC AAG GCA ATA ATG GGA 432
Met Ala Leu Tyr Asp Ile Ala Gln Lys Leu Pro Lys Ala Ile Met Gly
    130                 135                 140
CCG TCC TAT GGA TTC CAA TAC TCT CCC GCA GAA CGG GTC GAT TTC CTC 480
Pro Ser Tyr Gly Phe Gln Tyr Ser Pro Ala Glu Arg Val Asp Phe Leu
145                 150                 155                 160
CTC AAG GCT TGG GGA AGT AAA AAG GAC CCA ATG GGG TTC TCG TAT GAC 528
Leu Lys Ala Trp Gly Ser Lys Lys Asp Pro Met Gly Phe Ser Tyr Asp
                165                 170                 175
ACC CGC TGC TTT GAC TCC ACA GTC ACG GAG AGG GAC ATA AGA ACA GAA 576
Thr Arg Cys Phe Asp Ser Thr Val Thr Glu Arg Asp Ile Arg Thr Glu
            180                 185                 190
GAA TCC ATA TAT CAG GCT TGT TCC CTG CCT CAA GAG GCT AGA ACT GTC 624
Glu Ser Ile Tyr Gln Ala Cys Ser Leu Pro Gln Glu Ala Arg Thr Val
        195                 200                 205
ATA CAC TCG CTC ACT GAG AGA CTT TAC GTA GGA GGG CCC ATG ACA AAC 672
Ile His Ser Leu Thr Glu Arg Leu Tyr Val Gly Gly Pro Met Thr Asn
    210                 215                 220
AGC AAA GGG CAA TCC TGC GGT TAC AGG CGC TGC CGC GCA AGC GGT GTT 720
Ser Lys Gly Gln Ser Cys Gly Tyr Arg Arg Cys Arg Ala Ser Gly Val
225                 230                 235                 240

TTC ACC ACC AGC ATG GGG AAT ACC ATG ACA TGT TAC ATC AAA GCC CTT 768
Phe Thr Thr Ser Met Gly Asn Thr Met Thr Cys Tyr Ile Lys Ala Leu
                245                 250                 255
GCA GCG TGC AAA GCC GCA GGG ATT AAA GAC CCT ATC ATG CTG GTG TGT 816
Ala Ala Cys Lys Ala Ala Gly Ile Lys Asp Pro Ile Met Leu Val Cys
            260                 265                 270
GGA GAC GAC CTG GTC GTC ATC TCA GAG AGC CAG GGT AAC GAG GAG GAC 864
Gly Asp Asp Leu Val Val Ile Ser Glu Ser Gln Gly Asn Glu Glu Asp
        275                 280                 285
GAG CGA AAC CTG AGA GCT TTC ACG GAG GCT ATG ACC AGG TAT TCG GCC 912
Glu Arg Asn Leu Arg Ala Phe Thr Glu Ala Met Thr Arg Tyr Ser Ala
    290                 295                 300
CCT CCC GGT GAC CCT CCC AGA CCG GAA TAT GAC TTG GAG CTT ATA ACA 960
Pro Pro Gly Asp Pro Pro Arg Pro Glu Tyr Asp Leu Glu Leu Ile Thr
305                 310                 315                 320
TCC TGC TCC TCA AAT GTA TCG GTA GCG CTG GAC CCT CGG GGT CGC CGC 1008
Ser Cys Ser Ser Asn Val Ser Val Ala Leu Asp Pro Arg Gly Arg Arg
                325                 330                 335
CGG TAC TAC CTA ACC AGA GAC CCT ACC ACT CCA ATC TCC CGA GCT GCT 1056
Arg Tyr Tyr Leu Thr Arg Asp Pro Thr Thr Pro Ile Ser Arg Ala Ala
            340                 345                 350
TGG GAG ACA GTA AGA CAC TCC CCT GTC AAT TCT TGG CTG GGC AAC ATC 1104
Trp Glu Thr Val Arg His Ser Pro Val Asn Ser Trp Leu Gly Asn Ile
        355                 360                 365
ATC CAA TAC GCC CCT ACA ATC TGG GTC CGG ATG GTC ATA ATG ACC CAC 1152
Ile Gln Tyr Ala Pro Thr Ile Trp Val Arg Met Val Ile Met Thr His
    370                 375                 380

TTC TTT GCC ATA CTG TTG GCC CAG GAT ACT CTG AAC CAA AAT CTC AAT 1200
Phe Phe Ala Ile Leu Leu Ala Gln Asp Thr Leu Asn Gln Asn Leu Asn
385                 390                 395                 400
TTT GAG ATG TAC GGG GCA GTA TAT TCG GTC AAT CCA TTA GAC CTA CCG 1248
Phe Glu Met Tyr Gly Ala Val Tyr Ser Val Asn Pro Leu Asp Leu Pro
                405                 410                 415
GCC ATA ATT GAA AGG TTA CAT GGG CTT GAT GCC TTT TCA CTG CAC ACT 1296
Ala Ile Ile Glu Arg Leu His Gly Leu Asp Ala Phe Ser Leu His Thr
            420                 425                 430
TAC TCT CCC CAC GAA CTC TCG CGG GTG GCA GCG ACT CTC AGA AAA CTT 1344
Tyr Ser Pro His Glu Leu Ser Arg Val Ala Ala Thr Leu Arg Lys Leu
        435                 440                 445
GGA GCG CCT CCC CTT AGA GCG TGG AAG AGC CGG GCG CGT GCT GTG AGG 1392
Gly Ala Pro Pro Leu Arg Ala Trp Lys Ser Arg Ala Arg Ala Val Arg
    450                 455                 460
GCC TCA CTC ATC GCC CAA GGA GGG AGG GCA GCC ATT TGC GGC CGT TAC 1440
Ala Ser Leu Ile Ala Gln Gly Gly Arg Ala Ala Ile Cys Gly Arg Tyr
465                 470                 475                 480
CTC TTC AAC TGG GCG GTG AAG ACA AAG CTC AAA CTC ACT CCA TTG CCC 1488
Leu Phe Asn Trp Ala Val Lys Thr Lys Leu Lys Leu Thr Pro Leu Pro
                485                 490                 495
GAG GCG GCC CGC CTG GAT TTA TCC GGG TGG TTC ACC GTG GGC GCC GGC 1536
Glu Ala Ala Arg Leu Asp Leu Ser Gly Trp Phe Thr Val Gly Ala Gly
            500                 505                 510
GGG GGC GAC ATC TTT CAC AGC GTG TCG CGT GCC CGA CCC CGC TTA TTA 1584
Gly Gly Asp Ile Phe His Ser Val Ser Arg Ala Arg Pro Arg Leu Leu
        515                 520                 525

CTC CTT TGC CTA CTC CTA CTT AGT GTA GGG GTA GGC ATC TTT TTA CTC 1632
Leu Leu Cys Leu Leu Leu Leu Ser Val Gly Val Gly Ile Phe Leu Leu
    530                 535                 540
CCC GCT CGG TAGAGCGGCA ACCCCTAGCT ACACTCCATA GCTAGTGTCT 1681
Pro Ala Arg
545
TTGTTTTTTT TTTTT 1696

(2) INFORMATION FOR SEQ ID NO:7:
(i) SEQUENCE CHARACTERISTICS:
(A) LENGTH: 33 amino acids
(B) TYPE: amino acid
(D) TOPOLOGY: linear
(ii) MOLECULE TYPE: peptide
(xi) SEQUENCE DESCRIPTION: SEQ ID NO:7:

Gln Arg Lys Thr Lys Arg Asn Thr Asn Arg Arg Pro Gln Asp Val Lys
1               5                   10                  15
Phe Pro Gly Gly Gly Gln Val Val Gly Gly Val Tyr Leu Leu Pro Arg
            20                  25                  30
Arg

(2) INFORMATION FOR SEQ ID NO:8:
(i) SEQUENCE CHARACTERISTICS:
(A) LENGTH: 21 amino acids
(B) TYPE: amino acid
(D) TOPOLOGY: linear
(ii) MOLECULE TYPE: peptide
(xi) SEQUENCE DESCRIPTION: SEQ ID NO:8:

Asn Pro Lys Pro Gln Arg Lys Thr Lys Arg Asn Thr Asn Arg Arg Pro
1               5                   10                  15
Gln Asp Val Lys Phe
            20

(2) INFORMATION FOR SEQ ID NO:9:
(i) SEQUENCE CHARACTERISTICS:
(A) LENGTH: 40 amino acids
(B) TYPE: amino acid
(D) TOPOLOGY: linear
(ii) MOLECULE TYPE: peptide
(xi) SEQUENCE DESCRIPTION: SEQ ID NO:9:

Leu His Leu Asn Asp Arg Val Val Val Ala Pro Asp Lys Glu Ile Leu
1               5                   10                  15
Tyr Glu Ala Phe Asp Glu Met Glu Glu Cys Ala Ser Lys Ala Ala Leu
            20                  25                  30
Ile Glu Glu Gly Gln Arg Met Ala
        35                  40

(2) INFORMATION FOR SEQ ID NO:10:
(i) SEQUENCE CHARACTERISTICS:
(A) LENGTH: 15 amino acids
(B) TYPE: amino acid
(D) TOPOLOGY: linear
(ii) MOLECULE TYPE: peptide
(xi) SEQUENCE DESCRIPTION: SEQ ID NO:10:

Arg Arg Gly Pro Arg Leu Gly Val Arg Ala Thr Arg Lys Thr Ser
1               5                   10                  15

(2) INFORMATION FOR SEQ ID NO:11:
(i) SEQUENCE CHARACTERISTICS:
(A) LENGTH: 16 amino acids
(B) TYPE: amino acid
(D) TOPOLOGY: linear
(ii) MOLECULE TYPE: peptide
(xi) SEQUENCE DESCRIPTION: SEQ ID NO:11:

Arg Glu Arg Arg Gln Pro Ile Pro Lys Asp Arg Arg Ser Thr Gly Lys
1               5                   10                  15

(2) INFORMATION FOR SEQ ID NO:12:
(i) SEQUENCE CHARACTERISTICS:
(A) LENGTH: 20 amino acids
(B) TYPE: amino acid
(D) TOPOLOGY: linear
(ii) MOLECULE TYPE: peptide
(xi) SEQUENCE DESCRIPTION: SEQ ID NO:12:

Arg Gly Ser Arg Pro Thr Trp Gly Pro Thr Asp Pro Arg His Arg Ser
1               5                   10                  15
Arg Asn Leu Gly
            20

(2) INFORMATION FOR SEQ ID NO:13:
(i) SEQUENCE CHARACTERISTICS:
(A) LENGTH: 17 amino acids
(B) TYPE: amino acid
(D) TOPOLOGY: linear
(ii) MOLECULE TYPE: peptide
(xi) SEQUENCE DESCRIPTION: SEQ ID NO:13:

Pro Asp Lys Glu Ile Leu Tyr Glu Ala Phe Asp Glu Met Glu Glu Cys
1               5                   10                  15
Ala

(2) INFORMATION FOR SEQ ID NO:14:
(i) SEQUENCE CHARACTERISTICS:
(A) LENGTH: 14 amino acids
(B) TYPE: amino acid
(D) TOPOLOGY: linear
(ii) MOLECULE TYPE: peptide
(xi) SEQUENCE DESCRIPTION: SEQ ID NO:14:

Gln Trp Met Asn Arg Leu Ile Ala Phe Ala Ser Arg Gly Asn
1               5                   10

(2) INFORMATION FOR SEQ ID NO:15:
(i) SEQUENCE CHARACTERISTICS:
(A) LENGTH: 14 amino acids
(B) TYPE: amino acid
(D) TOPOLOGY: linear
(ii) MOLECULE TYPE: peptide
(xi) SEQUENCE DESCRIPTION: SEQ ID NO:15:

Leu Asp Ser Met Thr Glu Thr Glu Asp Asp Arg Glu Pro Ser
1               5                   10

(2) INFORMATION FOR SEQ ID NO:16:
(i) SEQUENCE CHARACTERISTICS:
(A) LENGTH: 16 amino acids
(B) TYPE: amino acid
(D) TOPOLOGY: linear
(ii) MOLECULE TYPE: peptide
(xi) SEQUENCE DESCRIPTION: SEQ ID NO:16:

Pro Thr Pro Gln Ala Pro Val Pro Pro Pro Arg Arg Arg Arg Ala lys
1               5                   10                  15

(2) INFORMATION FOR SEQ ID NO:17:
(i) SEQUENCE CHARACTERISTICS:
(A) LENGTH: 21 amino acids
(B) TYPE: amino acid
(D) TOPOLOGY: linear
(ii) MOLECULE TYPE: peptide
(xi) SEQUENCE DESCRIPTION: SEQ ID NO:17:

Ser Ala Lys Ser Leu Tyr Gly Phe Gly Ala Lys Glu Val Arg Ser Leu
1               5                   10                  15
Ser Arg Arg Ala Val
            20

(2) INFORMATION FOR SEQ ID NO:18:
(i) SEQUENCE CHARACTERISTICS:
(A) LENGTH: 24 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:18:

TACTGTCTTC ACGCAGAAAG CGTC 24

(2) INFORMATION FOR SEQ ID NO:19:
(i) SEQUENCE CHARACTERISTICS:
(A) LENGTH: 23 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:19:

GATCCAAGAA AGGACCCGGT CGT 23

(2) INFORMATION FOR SEQ ID NO:20:
(i) SEQUENCE CHARACTERISTICS:
(A) LENGTH: 25 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:20:

GAGCTCGGAT CCCGGAACCG GTGAGTACAC CGGAA 25

(2) INFORMATION FOR SEQ ID NO:21:
(i) SEQUENCE CHARACTERISTICS:
(A) LENGTH: 33 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:21:

GGTACCGTCG ACATTTACCC CGTCTTCCAG GAC 33

(2) INFORMATION FOR SEQ ID NO:22:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:22:

CCCGGGGATC CCGCCGACCT CATGGGGTAC AT     32

(2) INFORMATION FOR SEQ ID NO:23:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:23:

GGTACCGTCG ACAAATTCCC TGTTGCGTAA TT     32

(2) INFORMATION FOR SEQ ID NO:24:
(i) SEQUENCE CHARACTERISTICS:
(A) LENGTH: 33 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:24:

GAGCTCGGAT CCACGGTGTT AGGGTCCTGG AAG     33

(2) INFORMATION FOR SEQ ID NO:25:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:25:

GGTACCGTCG ACCGAGGATC ATGGTAAGAG TT     32

(2) INFORMATION FOR SEQ ID NO:26:
(i) SEQUENCE CHARACTERISTICS:
(A) LENGTH: 35 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:26:

CCCGGGGATC CCAAGAGCAC CAAAGTCCCT GTCGC 35

(2) INFORMATION FOR SEQ ID NO:27:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:27:

GGTACCGTCG ACACGCATAT GATGATGTCA TA 32

(2) INFORMATION FOR SEQ ID NO:28:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:28:

GAGCTCGGAT CCCCACCGGG GACCCTATCA CCTA 34

(2) INFORMATION FOR SEQ ID NO:29:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:29:

GGTACCGTCG ACCGGCATCA TAGCACTCGC AGAG 34

(2) INFORMATION FOR SEQ ID NO:30:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:30:

GAGCTCGGAT CCTCGTCAGG TGAAAGGCCG TCTG 34

(2) INFORMATION FOR SEQ ID NO:31:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:31:

GGTACCGTCG ACACCCGATC GTTCAGGTGT AG 32

(2) INFORMATION FOR SEQ ID NO:32:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:32:

GAGCTCGGAT CCCGAAATAC ATCGCCACGT GCAT 34

(2) INFORMATION FOR SEQ ID NO:33:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:34:

GGTACCGTCG ACCCTCATAT AAGATTTCCT TGTC 34

(2) INFORMATION FOR SEQ ID NO:34:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:34:

GAGCTCGGAT CCTCATTGGC CGCCTACACC TGAA 34

(2) INFORMATION FOR SEQ ID NO:35:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:35:

GGTACCGTCG ACTCAGACTC CACCACGTAG TG 32

(2) INFORMATION FOR SEQ ID NO:36:
(i) SEQUENCE CHARACTERISTICS:
(A) LENGTH: 33 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:36:

GAGCTCGGAT CCGATGAATA GACTGATCGC TTT 33

(2) INFORMATION FOR SEQ ID NO:37:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:37:

GGTACCGTCG ACGAGAAAAA TTCTGGAGCT GGTA 34

(2) INFORMATION FOR SEQ ID NO:38:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:38:

GAGCTCGGAT CCTGGCTCCT TCTCGTACGT AA 32

(2) INFORMATION FOR SEQ ID NO:39:
(i) SEQUENCE CHARACTERISTICS:
(A) LENGTH: 33 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:39:

GGTACCGTCG ACCGACGGAT CTGTCAGCAT GGA 33

(2) INFORMATION FOR SEQ ID NO:40:
(i) SEQUENCE CHARACTERISTICS:
(A) LENGTH: 30 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:40:

GAGCTCGGAT CCCTTCTTTC GGGATGAGGT 30

(2) INFORMATION FOR SEQ ID NO:41:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:41:

GGTACCGTCG ACTGAAAAGG TTGGCATCCA CCAT 34

(2) INFORMATION FOR SEQ ID NO:42:
(i) SEQUENCE CHARACTERISTICS:
(A) LENGTH: 31 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:42:

GAGCTCGGAT CCCCGTCTTT GAAGGCTATC T 31

(2) INFORMATION FOR SEQ ID NO:43:
(i) SEQUENCE CHARACTERISTICS:
(A) LENGTH: 33 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:43:

GGTACCGTCG ACACTCTGAT GGTACAGAAG GCT 33

(2) INFORMATION FOR SEQ ID NO:44:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:44:

GAGCTCGGAT CCGATGTGAC CCGGATTGAG TC 32

(2) INFORMATION FOR SEQ ID NO:45:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:45:

GGTACCGTCG ACGTCACCTT CTTTGCCCTC AGAG 34

(2) INFORMATION FOR SEQ ID NO:46:
(i) SEQUENCE CHARACTERISTICS:
(A) LENGTH: 34 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:46:

CCCGGGGATC CGGCCCTCAT AACACCATGT GGGC 34

(2) INFORMATION FOR SEQ ID NO:47:
(i) SEQUENCE CHARACTERISTICS:
(A) LENGTH: 32 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:47:

GGTACCGTCG ACTATAAGCT CCAAGTCATA TT 32

(2) INFORMATION FOR SEQ ID NO:48:

(i) SEQUENCE CHARACTERISTICS:

(A) LENGTH: 32 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:48:

CCCGGGGATC CAGCCTTCAC GGAGGCTATG AC 32

(2) INFORMATION FOR SEQ ID NO:49:

(i) SEQUENCE CHARACTERISTICS:

(A) LENGTH: 28 base pairs

(B) TYPE: nucleic acid

(C) STRANDEDNESS: single

(D) TOPOLOGY: linear

(ii) MOLECULE TYPE: DNA (genomic)

(xi) SEQUENCE DESCRIPTION: SEQ ID NO:49:

CTGCAGGTCG ACCCCCAAAA AAAAAAAA 28

## Description

### Background of the Invention

### Field of the Invention

The present invention relates to polypeptides which relate to non-A non-B hepatitis virus and DNAs encoding for the same. Further, the present invention relates to reagents for detecting anti non-A non-B hepatitis virus antibody and non-A non-B hepatitis virus gene.

### Description of the Related Art

Non-A non-B hepatitis is thought to amount to about 95 % of posttransfusive hepatitis at the present stage. Thus it has been urgently required to find out patients infected with non-A non-B hepatitis virus in order to exclude their blood from the blood to be transfused and to develop a vaccine for preventing this disease. Recently, Houghton et al. have reported the isolation of a gene of this virus [see European Patent Publication-A No. 318216 (published on May 31, 1989)] and marketed a reagent for detecting anti non-A non-B hepatitis virus antibody with the use of this recombinant viral antigen. Although it is expected that posttransfusive non-A non-B hepatitis would be prevented by finding blood wherein anti non-A non-B hepatitis virus antibody is present with this antibody detection reagent and excluding such blood from the blood to be transfused. However, this hepatitis occurs at an incidence of 5 to 7 % even after excluding the blood of anti non-A non-B hepatitis virus antibody positive donors. Thus, this disease has not been exterminated yet.

Indeed, one of the possible reasons therefor resides in the fact that the relation between the infection with this virus and the production of the antibody against this virus still remains unelucidated. However, another reason therefor resides in the finding that the nucleic acid sequence of the genome of this virus seemingly has a considerably high heterogeneity. This finding is based on the results of a number of experiments wherein c-DNA of this virus genome was cloned by the PCR method or the primer extension method based on the nucleic acid sequence reported by Houghton et al. [see Kubo et al., Nucleic Acid Research, 17, 10367 - 10372 (1989); Kato et al., Proc. Japan Acad., 65B, 219 - 223 (1989); Maeno et al., Nucleic Acid Research, 18, 2685 - 2689 (1990); Kato et al., Proc. Natl. Acad. Sci. USA, 87, 9524 - 9528 (1990)]. Accordingly, it may not be concluded that the diagnosis of non-A non-B hepatitis and the prevention against the onset of non-A non-B hepatitis caused by transfusion have been brought to completion and it has been thus required to develop a novel diagnostic method therefor including gene diagnosis.

Independently of Houghton et al. but at the same time, Terukatsu Arima, who is one of the present inventors, cloned one of epitopes in the core region of HCV (hepatitis C virus) by the immunoscreening method with the use of the serum of a human patient with non-A non-B hepatitis as an RNA source and an antibody source [see Arima et al., Gastroenterologia Japanica. 25, 218 - 222 (1990)].

### Disclosure of the Ivention

### Summary of the Invention

The present invention aims at providing a novel antigen polypeptide against non-A non-B hepatitis virus, DNA encoding the polypeptide and a diagnostic reagent for non-A non-B hepatitis with the use of the antigen polypeptide or the DNA.

Under the above-mentioned circumstances, the present inventors have started to study non-A non-B hepatitis virus. As a result of their extensive studies by using the plasmas of donors in Japan with a high s-GPT level, they have successfully isolated DNAs originating in novel non-A non-B hepatitis virus differing from the virus reported hitherto and confirmed the structures thereof. They have further found that non-A non-B hepatitis can be diagnosed by using these DNAs and polypeptides encoded thereby, thus completing the present invention.

Accordingly, the present invention relates to a non-A non-B hepatitis virus-constituting polypeptide represented by SEQ ID NO: 1, SEQ ID NO: 2 or SEQ ID NO: 3, and a polypeptide comprising a part of the amino acid sequence described in SEQ ID NO: 1, SEQ ID NO: 2 or SEQ ID NO: 3. That is, the present invention provides a polypeptide comprising at least 6 consecutive amino acids in the amino acid sequence represented by SEQ ID NO: 1, SEQ ID NO: 2 or SEQ ID NO: 3.

Further, the present invention relates to DNA (full length DNA) encoding the non-A non-B hepatitis virus-constituting polypeptide of the present invention, and DNA comprising a part of the base sequence of the above-mentioned full length DNA. That is, the present invention provides DNA comprising at least 10 consecutive bases in the base sequence represented by SEQ ID NO: 4, SEQ ID NO: 5 or SEQ ID NO: 6.

Furthermore, the present invention relates to a reagent for detecting anti non-A non-B hepatitis virus antibody by using a part of the above-mentioned non-A non-B hepatitis virus constituting polypeptide and confirming the presence of the anti non-A non-B hepatitis virus antibody in a sample by an immunological means, a reagent for detecting non-A non-B hepatitis virus gene by using a part of the above-mentioned full length DNA as a primer to amplify DNA originating in non-A non-B hepatitis virus and effecting the detection of the DNA, and a reagent for detecting non-A non-B hepatitis virus gene by using a part of the above-mentioned full length DNA as probe to effect hybridization with a DNA originating in non-A non-B hepatitis virus. That is, the present invention provides an immunochemical reagent for detecting anti non-A non-B hepatitis virus antibody in a biological sample which contains the polypeptide(s) of the present invention, and a reagent for detecting non-A non-B hepatitis virus gene which contains the DNA of the present invention.

In addition, the present invention relates to a polyclonal antibody or a monoclonal antibody against the above-mentioned non-A non-B hepatitis virus constituting polypeptide as an antigen, a reagent for detecting non-A non-B hepatitis virus antigen by using the above-mentioned antibody and confirming the presence of the non-A non-B hepatitis virus antigen in a sample by an immunochemical means, and a non-A non-B hepatitis vaccine produced by using the above-mentioned antigen polypeptide. That is, the present invention provides a polyclonal antibody against the polypeptide of the present invention as an antigen, a monoclonal antibody against the polypeptide of the present invention as an antigen, and a non-A non-B hepatitis virus vaccine produced by using the polypeptide of the present invention.

Further scope and applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Detailed Description of the Invention

Now, the present invention will be described in greater detail.

### (1) Preparation of RNA of non-A non-B hepatitis virus

As a material from which an RNA of non-A non-B hepatitis virus is extracted, the plasma of a donor with a high s-GTP level is usable. A viral fraction is collected by adding polyethylene glycol to the plasma and centrifuging it in accordance with a known method. From this viral fraction, the RNA may be extracted with, for example, a solution containing guanidine thiocyanate, a surfactant, a chelating agent and a reducing agent. That is, the purified RNA can be prepared by extracting the RNA from the viral fraction with the solution described above, and then with phenol, fractionating the extract with an organic solvent [see Chamezynski et al., Anal. Biochem., 162, 156 (1987)], and subjecting the fraction containing the RNA to density gradient ultracentrifugation.

### (2) Preparation and cloning of double stranded DNA

By using the obtained RNA as a template, a double-stranded DNA can be prepared by the conventional methods such as the method for synthesizing a cDNA with the use of, for example, a random primer, a reverse transcriptase and DNA polymerase [see Gubler, U., et al., Gene, 25, 263 (1983)].

In accordance with the conventional method, the double-stranded DNA thus obtained is integrated into a bacteriophage such as λzap and λgt11, Escherichia coli as a feeder bacterium is infected with the bacteriophage having the double-stranded DNA integrated thereinto, and then the Escherichia coli, i.e., the transformant thus obtained, is incubated. After screening, the target clone can be obtained. As an example of the method for integrating the double-stranded DNA into the phage vector, a method of Hyunh, T.V., et al. described in DNA Cloning, a practical approach, 1, 49 (1985) may be cited.

The screening of the target clone may be effected in accordance with a known method, for example, as follows. When the double-stranded DNA is integrated into a phage vector λgt11, E. coli Y1090 is infected with this vector and polypeptides contained in the plaques thus formed are transfered onto a nitrocellulose membrane. Next, this transfered membrane is subjected to a screening by an immunochemical means with the use of the serum of a patient with non-A non-B viral hepatitis as an antibody source.

However, it is difficult to analyze full sequence of the DNAs originating in non-A non-B hepatitis virus by this method. Therefore, the following method may be applied thereto.

First, an RNA is prepared from the fresh plasma of a patient who has been identified as having infection with non-A non-B hepatitis virus by the above-mentioned method. Then, DNAs are amplified by the conventional PCR method with the use of a single-strand DNA constructed by using the above-mentioned RNA as a template and an appropriate primer of a known structure, followed by subcloning. Thus, a DNA originating in non-A non-B hepatitis virus can be obtained. As the primer, an oligonucleotide, which is synthesized by appropriately selecting a well-maintained part on the basis of the known non-A non-B hepatitis virus DNA sequence, is usable. Usually, a fragment comprising 10 to 30 bases, which are derived from the non-A non-B hepatitis virus DNA, is used as the primer. The primers employed in the present invention are oligonucleotides synthesized based on the base sequences of HC-J8 [see Okamoto, H., et al., Virology, 188, 331 - 341 (1992)], HCV-J [see Kato, N., et al., Proc. Natl. Acad. Sci. USA, 87, 9524 - 9528 (1990)] and HC-J6 [see Okamoto, H., et al., J. Gen. Virol., 72, 2697 - 2704 (1991)] which have been already reported. Further, the partial base sequences of non-A non-B hepatitis virus was determined by experiments with the use of the primers described above and oligonucleotides each having a base sequence of the appropriately selected site in the partial base sequences of non-A non-B hepatitis virus thus determined, were synthesized and employed as primers.

The structure of a base sequence is identified by the Maxam-Gilbert method [see Maxam, A. M., and Gilbert, W., Proc. Natl. Acad. Sci. USA, 74, 560 (1977)] or the dideoxy method [see Sanger, F., Proc. Natl. Acad. Sci. USA, 74, 5463 (1977)].

### (3) Detection of anti non-A non-B hepatitis virus antibody and reagent therefor

By using the non-A non-B hepatitis virus-constituting polypeptide of the present invention or a part of the same as an epitope, the existence of the anti non-A non-B hepatitis virus antibody in a biological sample, for example, the serum of a patient can be examined by an immunochemical means, which enables the diagnosis of the infection with this virus. The term "epitope" means an antigen determinant of a polypeptide. It is well known that an epitope generally consists of at least 5 amino acids and a polypeptide consisting of 6 amino acid binds to an antibody [see European Patent Publication-A No. 138855 (published on May 2, 1985)]. As the epitope to be used herein, polypeptides comprising at least 3 to 5, preferably at least 8 to 10 and still more preferably at least 11 to 20 consecutive amino acids, which are based on the amino acid sequences described in SEQ ID NO: 1, SEQ ID NO: 2 and SEQ ID NO: 3, may be appropriately selected. Needless to say, even a polypeptide comprising about 20 or more amino acids may be used therefor.

As examples of the immunochemical assay methods, known ones such as the ELISA method, the Western blotting method and the agglutination method may be cited. From the viewpoints of convenience and utility, the ELISA method is preferred. The ELISA method may be effected in, for example, the following manner. A specimen is added to wells each comprising a solid phase having one or more epitopes adsorbed thereonto and reacted. After washing the wells, an enzyme-labeled anti-human IgG monoclonal antibody is added to the wells to react. After washing the wells, the amount of the binding enzyme is determined. Also cysteine may be added to the N-terminus of the epitope to thereby facilitate the adsorption of the epitope onto the solid phase.

As the substance for labeling the anti-human IgG antibody, any substances such as fluorescent substances, radioactive substances, bio- or chemiluminescent substances, electrochemical luminescent substances, pigments, enzymes and metals are usable, so long as it can be assayed. It is preferable to use enzymes and electrochemical luminescent substances therefor.

As the solid support for the epitope, microtiter plates, plastic beads, erythrocytes, gelatin particles, latex particles and magnetic particles may be cited and everyone are usable.

To select the epitope, it is preferable that site suitable as the epitope is confirmed by using the sera of a number of patients with non-A non-B hepatitis on the basis of the ELISA method.

Now, a particular example of the assay reagent of the present invention will be described. The assay reagent of the present invention is in the form of a kit which contains, as the essential component(s), one or more polypeptides each comprising the full length of a non-A non-B hepatitis virus-constituting polypeptide or a part thereof as its epitope, and at least one additional component(s) arbitrarily selected from among standard sera of patients with non-A non-B hepatitis virus (antibody), anti-human IgG antibody, enzymes and enzyme substrates. When this kit further contains a solid phase, this solid phase may be provided in a state of being coated with the polypeptide comprising the epitope. When it contains anti-human IgG antibody and an enzyme, these substances may be provided in a conjugated state. These kits also fall within the scope of the embodiment of the assay reagent of the present invention. Further, the kit may contain appropriate an antigen diluent, a reaction diluent, a solvent for a substrate or a reaction stopper solution for the convenience in the embodiment of the assay. These embodiments should not be considered to limit the present invention.

The specimen to be assayed is a so-called biological sample such as serum, plasma, spinal fluid, lymph, saliva and cells, though the specimen is not restricted thereto.

### (4) Preparation on of anti non-A non-B hepatitis virus polypeptide antibody

An antibody can be prepared by using, as the antigen, a polypeptide or a protein comprising the non-A non-B hepatitis virus polypeptide of the present invention or a part thereof. The polypeptide or the protein described above may be a complex of the non-A non-B hepatitis virus polypeptide of the present invention or a part thereof with a carrier protein. The complex may be prepared by using a coupling agent, if required. For example, glutaraldehyde, carbodiimide and maleimide active ester are usable therefor. As examples of the carrier protein, bovine serum albumin, thyroglobulin and hemocyanin may be cited. In a commonly employed case, the carrier protein is used in an amount of 1 to 5 times as much as the non-A non-B hepatitis virus polypeptide of the present invention or the part thereof.

As examples of the animal to be immunized, mouse, rat, rabbit and guinea pig may be cited. The inoculation may be effected by subcutaneous, intramuscular, intravenous or intraperitoneal administration. Before the inoculation, the antigen may be mixed with the complete Freund's adjuvant or the incomplete Freund's adjuvant and then administered. Usually, the inoculation is effected twice or more at intervals of 1 to 5 weeks. After sufficiently immunizing, the blood of the animal immunized is collected and the serum is separated to thereby give a polyclonal antibody. If necessary, purification may be effected to give the immunoglobulin fraction.

Antibody-producing cells are isolated as hybridomas which is prepared by fusing the antibody-producing cells obtained from the spleen or lymph nodes of the animal immunized with myeloma cells. As the myeloma cells, those originating in mouse, rat or human may be used. Although it is preferable that these myeloma cells have the same origin as that of the antibody-producing cells, those originating in different species can be fused with each other in some cases.

The cell fusion can be effected by a known method, for example, the method of Köhler and Milstein [see Nature, 256, 495 (1975)]. As examples of the fusion promoter, polyethylene glycol and Sendai virus may be cited. The cell fusion can be usually carried out by reacting the antibody-producing cells with the myeloma cells at a ratio by cell count of about 1 : 1 to 10 : 1 at a temperature of 20 to 40 °C (preferably 30 to 37 °C) for about 1 to 10 minutes by using polyethylene glycol (average molecular weight: 1,000 to 4,000) at a final concentration of about 20 to 50 %.

For screening the antibody-producing hybridomas, various immunochemical methods can be employed. For example, the ELISA (enzyme-linked immnosorbent assay) with the use of a microplate coated with an immune antigen, the EIA (enzyme immunoassay) method with the use of a microplate coated with antiimmunoglobulin antibody and the Western blotting method with the use of a nitrocellulose membrane having an antigen in the form of a solid phase are usable therefor.

With respect to the antibody-producing hybridomas in wells which are confirmed to produce the antibody by the above described immunochemical method, cloning is further effected by, for example, the limiting dilution analysis to thereby select an objective clone(s). In the selection of the objective hybridomas and the growing the selected hybridomas, a medium for animal cells containing 10 to 20 % of fetal calf serum (for example, PRMI 1640) to which HAT (hypoxanthine, aminopterin and thymidine) have been added is usually employed.

The clone thus obtained is intraperitoneally transplanted into a BALB/c mouse to which pristane has been administered. 10 to 14 days thereafter, the ascites fluid containing the monoclonal antibody at a high concentration is collected from the mouse immunized, and used as a material for the purification of the antibody. Also, a culture, which is obtained by incubating the clone thus obtained, can be used as a material for the purification of the antibody. To recover the monoclonal antibody, a known method for the purification of immunoglobulin may be used. For example, it can be easily achieved by ammonium sulfate fractionation, PEG fractionation, ethanol fractionation, use of an anion exchanger and affinity chromatography.

These antibodies make it possible to identify and assay non-A non-B hepatitis virus antigen in a biological sample by a known immunological method. Thus, these antibodies are usable as a diagnostic reagent for the non-A non-B hepatitis virus antigen.

### (5) Gene analysis on non-A non-B hepatitis virus

There have been reported 4 types of non-A non-B hepatitis virus including the above-mentioned one reported by Houghton et al. (HCV-1), one reported by Kato (HCV-J), one reported by Okamoto et al. (HC-J6) and the virus of the present invention. As these facts clearly indicate, it is known that there are several subtypes of the non-A non-B hepatitis virus.

As discussed above, it is difficult to identify and distinguish these viral subtypes by immunologically assaying an antibody against the virus or an antigen polypeptide of this virus in a biological sample, though this method is useful for the diagnosis of the infection with this virus.

The DNA described in SEQ ID NO: 4 corresponds to a part of a region which encodes the structural protein of the virus, while the DNAs described in SEQ ID NO: 5 and SEQ ID NO: 6 each corresponds to a part of a region which encodes the non-structural protein thereof. A comparison of the DNA base sequence of each subtype of non-A non-B hepatitis virus with the DNA base sequences of the present invention shows a homology of 70 to 80 %. This fact indicates that the subtypes of the non-A non-B hepatitis virus can be distinguished from each other by the gene analysis with the use of a suitable oligonucleotide as a primer on the basis of the DNA base sequence of the corresponding part of the non-A non-B hepatitis virus.

The oligonucleotide to be used as the primer comprises at least 6 bases which is contained in the DNA encoding the polypeptide of the present invention. The number of the bases derived from the DNA encoding the polypeptide of the present invention in the oligonucleotide is preferably at least 8, still more preferably at least 10 to 12, and most preferably from 15 to 25. That is, the base sequnce of the primer may be appropriately selected based on the base sequence (DNA sequence) of the virus of each subtype.

The subtype of the non-A non-B hepatitis virus can be identified in the conventional manner by using an RNA prepared from a biological sample such as a plasma specimen as a template, amplifying a DNA by the RT-PCR method with the use of an appropriate antisense primer, a sense primer, a reverse transcriptase and a thermostable DNA polymerase and then analyzing the DNA. The type of the virus can be identified by examining whether the DNA of the given size has been amplified or not by polyacrylamide gel or agarose gel electrophoresis. Further, the confirmation of the subtype can be made by using, as a probe, a labeled synthetic oligonucleotide which is derived from the virus of each subtype and of which sequence is present between two primers employed.

The structure of the amplified DNA may be analyzed by directly analyzing the base sequence from the PCR product. If necessary, it is recommended to integrate the DNA into an appropriate vector and then introduce this vector into an appropriate host followed by replication of the DNA. Thus the base sequence of the DNA can be analyzed.

Further, the use of the non-A non-B hepatitis virus polypeptide of the present invention makes it possible to produce a vaccine which is usable as a medicine for preventing or treating non-A non-B hepatitis.

The use of the DNA of the present invention, the RNA which is complementary thereto or an antigen polypeptide of the present invention enables the diagnosis of non-A non-B hepatitis, which makes it possible to prevent the infection with this virus via, for example, transfusion and to develop a vaccine and an antiviral agent therefor.

### Examples

The present invention will now be described in greater detail with reference to the following Examples which should not be considered to limit the scope of the present invention.

### Example 1

### Preparation of nor-A non-B hepatitis virus RNA from plasma

The plasmas of donors who had been judged as strongly positive for C100-3 antibody (2.00 ≧) by using an antibody detection reagent comprising a so-called viral antigen of Houghton et al. and the plasmas of patients with various hepatitises were subjected to the identification of subtypes in accordance with the method disclosed in International Publication No. WO92/18532 (published on Oct. 29, 1992), and corresponding thereto of European Patent Publication-A No. 580754 (published on Feb. 2, 1994). Then, plasmas containing only the virus 2-22 as described in the International Publication No. WO 92/18532 were employed as a starting material to prepare the RNA.

To 1 ml of the plasma of a donor was added polyethylene glycol 4000 in such a manner as to give a final concentration of 4 %. After dissolution by stirring at 4 °C for 90 minutes, the solution thus obtained was centrifugated at 14,000 rpm for 10 minutes to give a precipitate. To this precipitate were added 200 µl of 6 M guanidine isothiocyanate, 2.1 µl of β-mercaptoethanol, 5 µl of yeast t-RNA (10 mg/ml), 22 µl of 2 M sodium citrate (pH 4.0) and 350 µl of phenol/chloroform (5 : 2). The resulting mixture was stirred at room temperature for 1 minute, allowed to stand at 0 °C for 15 minutes and then centrifuged at 14,000 rpm for 10 minutes. The aqueous layer of this reaction mixture was taken out and 350 µl of phenol/chloroform (5 : 2) was added thereto. After stirring at room temperature for 1 minute, the mixture was allowed to stand at 0 °C for 15 minutes and centrifuged at 14,000 rpm for 10 minutes. The aqueous layer of the reaction mixture was taken out and extracted with phenol/chloroform again. To the aqueous layer taken therefrom was added 2-propanol in the same amount as the aqueous layer and the mixture thus obtained was allowed to stand at -20 °C for 12 hours or longer. Then, it was centrifuged at 14,000 rpm for 20 minutes to give a precipitate. To this precipitate were added 85 µl of 4 M guanidine isothiocyanate, 0.7 µl of β-mercaptoethanol, 15 µl of 2 M sodium citrate (pH 4.0) and 100 µl of 2-propanol to effect isopropanol precipitation to thereby prepare an RNA.

### Example 2

### Gene amplification by RT-PCR method and structural analysis

20 µl of the RNA solution thus obtained was centrifuged at 14,000 rpm for 10 minutes to thereby give a precipitate. This precipitate was washed with 70 % ethanol and dried. Next, 25 µl of a reagent mixture [10 mM Tris HCl (pH 8.3), 50 mM KCl, 4 mM MgCl₂, 1 mM DTT, 0.001 % gelatin], 2 µl of a solution of an antisense primer (10 µM) in the 3'-downstream side as will be described hereinafter and 10 µl of a solution of a mixture of 4 deoxynucleotides [1.25 mM portions of dATP, dGTP, dCTP and dTTP] were added thereto to give 37 µl of a solution.

This solution was heated successively at 70 °C for 1 minute and at 55 °C for 1 minutes and then cooled to 0 °C. After adding 2 µl (80 U) of RNase inhibitor (mfd. by Promega) and 1 µl (200 U) of a reverse transciptase (mfd. by BRL), the mixture thus obtained was reacted at 37 °C for 30 minutes. Then, it was heated to 95 °C and maintained at that temperature for 5 minutes to thereby inactivate the enzyme. To 4 µl of this reaction mixture was added 44.2 µl of a PCR solution [10 mM Tris HCl (pH 8.3), 50 mM KCl, 1.5 mM MgCl₂, 0.001 % gelatin, dNTPs mixture each 125 µM] . Then, 0.5 µl of a solution of a primer (10 µM) having a base sequence of the sense strand in the 5'-upstream side of the region to be subjected to the gene amplification and 0.3 µl of a solution of a primer (10 µM) having a base sequence of the antisense strand in the 3'-downstream side of the above-mentioned region were added to thereby give a solution of 49 µl in the total volume.

The primers employed herein were those which had been synthesized on the basis of the DNA sequences of HC-J8, HC-J6, HCV-J and those identified during the experiment. The original viruses and base position numbers of these sense primers and antisense primers and sequences of which structures are identified by using these primers are listed in Tables 1 and 2 in accordance with the base numbers given in the sequence listing. In these Tables, J, J6 and J8 mean respectively HCV-J, HC-J6 and HC-J8 as described above, while Seq. 4, Seq. 5 and Seq. 6 mean respectively SEQ ID NO: 4, SEQ ID NO: 5 and SEQ ID NO: 6 which will be described hereinafter. To effect cloning, a sequence recognized by restriction enzymes Sma I and Bam HI was added to the 5'-side of some sense primers, while another sequence recognized by restriction enzymes Sal I and Pst I was added to the 3'-side of some antisense primers.

**Table 1**

| Primer | Origin of the primer and base number of the sequence used | | Base number of the sequence identified with the primer | |
|---|---|---|---|---|
| Sense | J, | 44-67 | | |
| Antisense | J, | 192-170 | Seq. 4, | 1-102 |
| Sense | J8, | 151-173 | | |
| Antisense | Seq.4, | 751-731 | Seq. 4, | 95-730 |
| Sense | J, | 719-739 | | |
| Antisense | J8, | 847-828 | Seq. 4, | 673-748 |
| Sense | Seq. 4, | 719-740 | | |
| Antisense | J8, | 1344-1325 | Seq. 4, | 741-1245 |
| Sense | J6, | 4058-4080 | | |
| Antisense | J8, | 4299-4281 | Seq. 5, | 1-198 |
| Sense | Seq. 5, | 130-150 | | |
| Antisense | J8, | 4924-4902 | Seq. 5, | 150-819 |
| Sense | Seq. 5, | 778-800 | | |
| Antisense | Seq. 5, | 1348-1328 | Seq. 5, | 800-1326 |
| Sense | J8, | 5278-5299 | | |
| Antisense | J8, | 5463-5442 | Seq. 5, | 1218-1359 |

**Table 2**

| Primer | Origin of the primer and base number of the sequence used | | Base number of the sequence identified with the primer | |
|---|---|---|---|---|
| Sense | J8, | 5398-5419 | | |
| Antisense | J8, | 6169-6150 | Seq.5, | 1338-2067 |
| Sense | Seq. 5, | 2017-2037 | | |
| Antisense | J8, | 6721-6700 | Seq. 5, | 2037-2617 |
| Sense | Seq. 5, | 2560-2579 | | |
| Antisense | Seq. 5, | 2808-2789 | Seq. 5, | 2579-2787 |
| Sense | J8, | 6768-6787 | | |
| Antisense | J8, | 7050-7029 | Seq. 5, | 2706-2946 |
| Sense | Seq. 5, | 2895-2915 | | |
| Antisense | J8, | 7161-7141 | Seq. 5, | 2915-3058 |
| Sense | J8, | 7059-7078 | | |
| Antisense | Seq. 6, | 26-5 | Seq. 5, | 2988-3633 |
| Sense | J8, | 7691-7013 | | |
| Antisense | J8, | 8756-8637 | Seq. 6, | 1-937 |
| Sense | J, | 8597-8617 | | |
| Antisense | J, | 9412-9401 | Seq. 6, | 900-1696 |

After adding l µl of Taq polymerase (0.5 U, mfd. by Perkin-Elmer Cetus) to the solution described above, denaturation (94 °C, 1 minute), annealing (45 °C, 1 minute) and polymerization (72 °C, 3 minutes) were repeated 30 times. To 4 µl of the reaction mixture thus obtained were further added 90 µl of the above-mentioned PCR solution, 1 µl of a solution of the sense primer, 1 µl of a solution of the antisense primer and 2 µl of Taq polymerase and then the DNA was amplified by repeating denaturation (94 °C, 1 minute), annealing (45 °C, 1 minute) and polymerization (72 °C, 3 minutes) 30 times.

Now the cloning of the gene product amplified by the RT-PCR method will be illustrated.

The amplified gene fragments were electrophoresed on a polyacrylamide gel (4 to 5 %) and stained with ethidium bromide. Then, a DNA of the target length was recovered. This DNA was cleaved with restriction enzymes Bam HI and Sal I and the resultant fragments were inserted into the Bam HI - Sal I site of phage vectors M13mp18 and M13mp19. Then, the gene was cloned by the conventional manner and the base sequence was analyzed.

The base sequence was identified by the dideoxy termination method with the use of M13 phages in the conventional manner. Based on the base sequences of the obtained clones, the overlapping parts were checked up. Thus, the base sequences represented by SEQ ID NO: 4, SEQ ID NO: 5 and SEQ ID NO: 6 have been identified. Based on these sequences, it has been deduced that the amino acid sequences encoded thereby are those described in SEQ ID NO: 1, SEQ ID NO: 2 and SEQ ID NO: 3. SEQ ID NO: 1 corresponds to the structural region, while both SEQ ID NO: 2 and SEQ ID NO: 3 correspond to the nonstructural regions.

### Example 3

### Confirmation of epitope site

It was confirmed which site of the amino acid sequence of the non-A non-B hepatitis virus-constituting polypeptide of the present invention described in SEQ ID NO: 1, SEQ ID NO: 2 or SEQ ID NO: 3 would be suitable as an epitope. First, polypeptides listed in Table 3 were synthesized based on the amino acid sequence of the present invention and screened.

**Table 3**

| Peptide | Location of amino acid sequence in sequence listing | | Positive ratio (%) in plasmas of positive donors (n = 130) |
|---|---|---|---|
| CL | SEQ ID. NO:1, | 8-40 | 100 |
| M1 | SEQ ID. NO:1, | 4-24 | 94.6 |
| M2aE | SEQ ID. NO:1, | 39-53 | 62.3 |
| M2cE | SEQ ID. NO:1, | 59-74 | 52.3 |
| M3E | SEQ ID. NO:1, | 101-120 | 31.5 |
| 57 | SEQ ID. NO:2, | 443-482 | 84.6 |
| M11E | SEQ ID. NO:2, | 453-469 | 58.5 |
| M51E | SEQ ID. NO:2, | 671-684 | 24.6 |
| M15E | SEQ ID. NO:2, | 1009-1022 | 3.1 |
| M17E | SEQ ID. NO:2, | 1071-1086 | 24.6 |
| M32E | SEQ ID. NO:3, | 52-72 | 17.7 |

First, each 5.0 µg/ml solution containing a synthetic peptide was pipetted into a well of a microplate in an amount of 100 µl to thereby coat the well therewith. After blocking with 100 µl of a blocking buffer (10 mM PBS, 1 % BSA, 0.1 % Tween 20, pH 7.4), 100 µl portions of a reaction solution (50 mM Tris, 1 mM EDTA, 0.1 % NaN₃, 10 % NRS, pH 8.0) were added to the wells. Next, 20 µl portions of the plasma of a donor was added thereto and the microplate was incubated at 37 °C for 60 minutes. After the incubation, the wells were washed with a washing buffer (10 mM PBS, 0.1 % BSA, 0.1 % Tween 20, pH 7.4) and then 100 µl portions of alkaline phosphatase-labeled anti-human IgG antibody were added to the wells. After incubating at 37 °C for 60 minutes and washing with the washing buffer, 100 µl portions of a solution of an eznyme substrate (p-nitrophenyl phosphate 1.0 mg/ml, carbonate buffer, pH 9.8) was added to the wells. After reacting at 37 °C for 30 minutes, the absorbances of the reaction solutions in the wells were measured at a wavelength of 405 nm.

In the right column of Table 3, the positive ratios to the plasmas of donors (130 specimens), who had been judged as positive with a reagent of the second generation, i.e., a second generational anti HCV antibody detection reagent, thus determined are given. As Table 3 shows, it is confirmed that the positive ratios determined by using the peptides CL, M1 and 57 are respectively 100 %, 94.6 % and 84.6 %. Thus it is proved that these 3 peptides are effective as an epitope. Namely, this result means that a reagent comprising a part of the amino acid sequence of the present invention as the epitope is effective in the diagnosis of non-A non-B hepatitis. Since a novel epitope is employed in the diagnostic reagent of the present invention, it is expected that the diagnostic reagent would exert a usefulness, which has never been achieved by the conventional ones, through the further accumulation of clinical test data. The amino acid sequences of the peptides CL, M1 and 57 are represented by SEQ ID NO: 7, SEQ ID NO: 8 and SEQ ID NO: 9, respectively.

## Claims

1. A polypeptide comprising at least 6 consecutive amino acids in the amino acid sequence represented by SEQ ID NO: 1, SEQ ID NO: 2 or SEQ ID NO: 3.

2. The polypeptide as claimed in claim 1, which is a non-A non-B hepatitis virus-constituting polypeptide represented by SEQ ID NO: 1, SEQ ID NO: 2 or SEQ ID NO: 3.

3. The polypeptide as claimed in claim 1, which is represented by SEQ ID NO: 7, SEQ ID NO: 8 or SEQ ID NO: 9.

4. DNA comprising at least 10 consecutive bases in the base sequence represented by SEQ ID NO: 4, SEQ ID NO: 5 or SEQ ID NO: 6.

5. The DNA as claimed in claim 4, which encodes the polypeptide represented by SEQ ID NO: 1, SEQ ID NO: 2 or SEQ ID NO: 3, and is represented by SEQ ID NO: 4, SEQ ID NO: 5 or SEQ ID NO: 6.

6. An immunochemical reagent for detecting anti non-A non-B hepatitis virus antibody in a biological sample which contains the polypeptide(s) as claimed in claim 1.

7. The immunochemical reagent for detecting anti non-A non-B hepatitis virus antibody as claimed in claim 6, which contains one or more polypeptide(s) selected from the group consisting of polypeptides represented by SEQ ID NO: 7, SEQ ID NO: 8 and SEQ ID NO: 9.

8. A reagent for detecting non-A non-B hepatitis virus gene which contains the DNA as claimed in claim 4.

9. A polyclonal antibody against the polypeptide as claimed in claim 1 as an antigen.

10. A monoclonal antibody against the polypeptide as claimed in claim 1 as an antigen.

11. A non-A non-B hepatitis virus vaccine produced by using the polypeptide as claimed in claim 1.
